# EUROPEAN PATENT SPECIFICATION

(11) **EP 3 206 678 B1**
(45) Date of publication and mention of the grant of the patent: **11.12.2019**
(21) Application number: 15820308.3
(22) Date of filing: 14.10.2015
(51) Int. Cl.: A61K 31/23, A61P 3/04, C07C 219/30, A61K 9/00, A61K 9/06

(54) **BODY SCULPTING**
KÖRPERMODELLIERUNG
FAÇONNAGE DU CORPS

(30) Priority: 14.10.2014 NL 2013634
(43) Date of publication of application: 23.08.2017
(73) Proprietor: Sculpt B.V., 9713 GX Groningen (NL)
(72) Inventor: CREMERS , Thomas, 9713 GX Groningen (NL); FLIK, Gunnar, 9713 GX Groningen (NL); FRIJLINK, Henderik Willem, 9713 AV Groningen (NL); WOERDENBAG, Herman Johan, 9713 AV Groningen (NL)
(74) Representative: V.O.
(86) International application number: PCT/NL2015/050722
(87) International publication number: WO 2016/060564

(56) References cited:
- EP-A1- 0 108 822
- EP-A2- 0 120 165
- WO-A1-2005/108381
- WO-A1-2007/071452
- WO-A1-2007/109882
- WO-A2-2010/138770
- DE-A1- 2 341 876
- US-A- 4 085 270
- US-A1- 2004 235 922
- US-A1- 2007 014 843
- DATABASE CA [Online] CHEMICAL ABSTRACTS SERVICE, COLUMBUS, OHIO, US; PENG, YUANHONG ET AL: "Method for preparing transdermally absorbable synephrine derivatives", XP002754820, retrieved from STN Database accession no. 2013:793417 -& CN 103 113 244 A (HUNAN XINLI BIOLOG SCIENCE & TECHNOLOGY CO LTD; UNIV CHONGQING) 22 May 2013 (2013-05-22)
- GREENWAY F L ET AL: "REGIONAL FAT LOSS FROM THE THIGH IN OBESE WOMEN AFTER ADRENERGIC MODULATION", CLINICAL THERAPEUTICS, EXCERPTA MEDICA, PRINCETON, NJ, US, vol. 9, no. 6, 1 January 1987 (1987-01-01) , pages 663-669, XP009000026, ISSN: 0149-2918
- AHMED: "Evaluation of Stereoselective transdermal transport and concurrent cutaneous hydrolysis of ...", PHARMACEUTICAL RESEARCH, 1 January 1996 (1996-01-01), pages 1524-1529, XP055162819,
- SUM F W ET AL: "Prodrugs of CL316243: a selective beta3-adrenergic receptor agonist for treating obesity and diabetes", BIOORGANIC & MEDICINAL CHEMISTRY LETTERS, PERGAMON, AMSTERDAM, NL, vol. 9, no. 14, 19 July 1999 (1999-07-19), pages 1921-1926, XP004171610, ISSN: 0960-894X, DOI: 10.1016/S0960-894X(99)00316-9
- JOSEF WAGNER ET AL: "Prodrugs of etilefrine: Synthesis and evaluation of 3'-(O-acyl) derivatives", JOURNAL OF PHARMACEUTICAL SCIENCES, vol. 69, no. 12, 1 December 1980 (1980-12-01), pages 1423-1427, XP55252712, WASHINGTON, US ISSN: 0022-3549, DOI: 10.1002/jps.2600691219
- SVENSSON ET AL: "Toward lung-selective beta2-adrenergic bronchodilator prodrugs", ADVANCED DRUG DELIVERY REVIEWS, ELSEVIER, AMSTERDAM, NL, vol. 14, no. 2-3, 1 June 1994 (1994-06-01) , pages 319-330, XP023861829, ISSN: 0169-409X, DOI: 10.1016/0169-409X(94)90048-5 [retrieved on 1994-06-01]
- ANDREA GUALANDI ET AL: "Pyrrole Macrocyclic Ligands for Cu-Catalyzed Asymmetric Henry Reactions", THE JOURNAL OF ORGANIC CHEMISTRY, vol. 76, no. 9, 6 May 2011 (2011-05-06), pages 3399-3408, XP55253506, US ISSN: 0022-3263, DOI: 10.1021/jo200318b
- SLOAN KENNETH B ET AL: "Designing for topical delivery: prodrugs can make the difference", MEDICINAL RESEARCH REVIEWS, NEW YORK, NY, US, vol. 23, no. 6, 1 November 2003 (2003-11-01), pages 763-793, XP002608795, ISSN: 0198-6325, DOI: 10.1002/MED.10048 [retrieved on 2003-08-20]

## Description

The present invention relates to drugs or prodrugs that are used for locally modulating subcutaneous fat tissue, and to pharmaceutical compositions comprising these drugs or prodrugs.

Oral or topical administration of adrenergic receptor agonists and/or adrenergic receptor antagonists is known for various purposes, among which fat reduction. However, none of the known methods disclose the use of adrenergic receptor agonists or adrenergic receptor antagonists, or prodrugs thereof, with a high octanol/water partition coefficient to locally target subcutaneous fat tissue. A drawback of treating mammalian subjects with adrenergic receptor agonists or adrenergic receptor antagonists is that systemic concentrations easily become too high, which results in significant side effects.

The present invention relates to a pharmaceutical composition for topical administration comprising a prodrug for an agonist and/or an antagonist for an adrenergic receptor, wherein the prodrug is an ester, which prodrug comprises said agonist or antagonist and a hydrolyzable moiety, wherein the prodrug has an octanol/water partition coefficient of at least 0, for use in a therapeutic method of shaping a mammalian body by modulation of subcutaneous fat tissue.

A mammal, in the present context, is any mammalian animal, such as for example a dog, cat, horse, mouse, rat or human. Preferably, the mammal is a human.

It is an advantage of the present invention that the prodrug of the invention, when topically administered, has a high affinity for fat tissue.

Topical administration means that the prodrug is applied to the skin of a mammal, and penetrates the skin. Alternatively, topical administration in the present context may mean that the prodrug is administered by microinjection or by iontoforesis. As such, topical administration in the present context is the same as transdermal application. The prodrug of the invention preferably targets the subcutaneous fat tissue that is present at the site of topical administration. This results in local accumulation of the prodrug in subcutaneous fat tissue at the application site, and essentially avoids systemic absorption.

The absorption of the prodrug of the invention into the subcutaneous fat tissue results in an increased concentration of the agonist and/or antagonist in the local fat tissue, because the prodrug is hydrolyzed by the action of locally present endogenous enzymes, so that the adrenergic receptor agonist and/or the adrenergic receptor antagonist is released from the prodrug inside the subcutaneous fat tissue. Enzymes which are capable of releasing the agonist or antagonist from the prodrug include for example lipase, esterase, paraoxonase, carboxylesterase, acetylcholinesterase, cholinesterase, biphenyl hydrolase, alkaline phosphatase, amidase, transpeptidase, CYP 450, trypsin, chymotrypsin, elastase, carboxypeptidase, aminopeptidase. Preferably, the enzyme is a lipase enzyme.

Whether an endogenous enzyme, preferably present in subcutaneous fat tissue, is capable of releasing the agonist or antagonist from the prodrug can be tested by subjecting the prodrug to the enzyme or tissue in question, and determining whether drug is released from the prodrug by a suitable analytic technique, such as for example UV-Vis spectrometry, mass spectometry or gas- or liquid chromatography.

The high affinity for fat tissue of the prodrug has the advantage that the concentration of the agonist or antagonist in the local fat tissue can be increased multifold, essentially without affecting the systemic concentration of the agonist or antagonist. This avoids the side effects associated with administration of adrenergic receptor agonists or adrenergic receptor antagonists of the prior art.

The increased potential concentration in fat tissue enhances the natural effect of the agonist or antagonist on subcutaneous fat tissue relative to the case where no additional agonists or antagonists are absorbed into the fat tissue. This allows for local shaping of a mammalian body, because the agonist or antagonist interacts with an adrenergic receptor, which controls fat degradation or fat build-up in subcutaneous fat tissue.

In addition, the topical administration route avoids the hepatic first-pass effect, contributing to the locally increased concentrations in subcutaneous fat tissue responsible for the shaping.

The pharmaceutical composition of the invention comprises an agonist or an antagonist for the adrenergic receptor, with a octanol/water partition coefficient of at least 0, preferably at least 1, more preferably at least 2, more preferably at least 2.3, even more preferably at least 2.5, even more preferably at least 3. The octanol/water partition coefficient is a measure for the lipophilicity of a compound, well known to the skilled person.

The octanol/water partition coefficient can be determined by the shake-flask method, which consists of dissolving some of the agonist, antagonist or prodrug as the solute in question in a mixture of equal amounts of octanol and water, and then measuring the concentration of the solute in each solvent. The octanol/water partition coefficient is calculated by the ratio of the concentration of the solute in octanol, relative to the concentration in water, and expressed as ¹⁰log value. The octanol/water partition coefficient is also referred to as logP. The octanol/water partition coefficient can accurately be predicted by calculation in standard chemical software, such as for example ChemSketch™ or ChemDraw™.

An octonal/water partition coefficient of 0 means the solute is present at equal concentration in the octanol and water phases, whereas an octanol water partition coefficient of 2 means that the concentration of the solute in octanol is 100 times the concentration of the solute in water.

Two types of adrenergic receptors exist, alpha adrenergic receptors and beta adrenergic receptors.

Stimulation of an alpha adrenergic receptor by an agonist ("alpha-agonist") has the effect that lipase activity is suppressed. Suppression of lipase activity has the effect that triglyceride hydrolysis is reduced relatively to triglyceride production. This has the effect of increasing the amount of triglyceride in tissue, thereby increasing the quantity of subcutaneous fat tissue. Inhibition of an alpha adrenergic receptor by an antagonist ("alpha-antagonist") has the opposite effect, and decreases the quantity of subcutaneous fat tissue.

Stimulation of a beta adrenergic receptor by an agonist ("beta-agonist") has the effect that lipase activity is increased, so that hydrolysis of triglycerides is increased relative to triglyceride production. As the amount of triglycerides decreases, the quantity of subcutaneous fat tissue decreases. Inhibition of a beta adrenergic receptor by an antagonist ("beta-antagonist") has the opposite effect, and results in increasing the quantity of subcutaneous fat tissue.

It follows that an increased presence in subcutaneous fat tissue of either a beta adrenergic receptor agonist or an alpha adrenergic receptor antagonist has the effect of decreasing the quantity of subcutaneous fat tissue. An increased presence in subcutaneous fat tissue of either a beta adrenergic receptor antagonist or an alpha adrenergic receptor agonist has the effect of increasing the quantity of subcutaneous fat tissue.

The beta adrenergic receptor may be for example a beta-1, a beta-2 or a beta-3 receptor, but preferably, it is a beta-3 receptor. The alpha adrenergic receptor is preferably an alpha-2 adrenergic receptor.

An agonist is a compound which activates (stimulates) an adrenergic receptor, and an antagonist is a compound which inhibits an adrenergic receptor; it can be tested whether a compound is an agonist or an antagonist for an adrenergic receptor by studying the effect of the compound on a suitable second messenger signal.

An agonist for a beta adrenergic receptor (also called a beta adrenergic receptor agonist, or "beta-agonist"), for the context of the present invention, is any compound which activates a beta adrenergic receptor, preferably a beta-3 receptor. Alternatively or additionally, it is any compound that elevates glycerol according to the methods described in the examples.

Whether a compound is an agonist of a beta adrenergic receptor, preferably the beta-3 adrenergic receptor, can be tested by measuring stimulation or inhibition of a second messenger signal upon incubation of compounds with suitable receptor containing material. A suitable second messenger signal is for instance cyclic AMP (cAMP). In the case of cAMP as second messenger, an increase in the quantity of cAMP indicates stimulation of the beta adrenergic receptor. Conversely, a decrease in the quantity of cAMP indicates inhibition of the beta adrenergic receptor.

In the case of an alpha adrenergic receptor, an agonist decreases the quantity of cAMP, and an inhibitor increases the quantity of cAMP.

An agonist may be a partial or a full agonist. Full agonists are agonists that upon binding to the receptor, display maximum activation of that receptor. Partial agonists are agonists that upon binding to the receptor, only display partial activation, relative to the activation achieved with a full agonists. Partial and full agonists can be distinguished by activating a receptor with a full agonist and determining the magnitude of its maximum activation by recording activation based on a suitable second messenger signal as described above. If the second messenger signal attained with a sample agonist is as high as that attained with the full agonist, the sample agonist is a full agonist. If the second messenger signal is lower, it is a partial agonist. For the present context, both partial and full agonists are considered agonists, but full agonists are preferred.

Agonists can increase or decrease the second messenger signal directly or indirectly. Direct activation means that the agonist increases the second messenger signal by a direct molecular interaction between the agonist and the adrenergic receptor. Indirect activation means that the agonist increases the second messenger signal by molecular interaction with a species which is not an adrenergic receptor. Alternatively, indirect activation occurs via elevation of a naturally occurring beta agonist such as norepinephrine through mechanisms such as re-uptake inhibition.

Whether a compound is an indirect agonist of an adrenergic receptor can be tested by norepinephrine uptake assays, or phosphodiesterase inhibition assays. Affinities (IC50) typically range between 0.05 nM- 200 nM. Indirect agonists for the adrenergic receptor include but are not limited to NE (norepinephrine) uptake inhibitors, NE releasers, phosphodiesterase inhibitors, adenyl cyclase activators and neurotransmission modulators. Preferably, an indirect adrenergic receptor agonist of the invention is fenfluramine, forskolin, caffeine, theophylline, rimonabant or amphetamine, most preferably amphetamine, forskolin or caffeine.

Examples of adrenergic receptor agonists include beta agonists and alpha agonists. Beta agonists are preferred. Among beta agonists, beta-3 agonists are preferred. Among alpha agonists, alpha-2 agonists are preferred. Among all agonists, beta-3 agonists and alpha-2 agonists are much preferred, and most preferred are beta-3 agonists.

Examples of suitable beta agonists (also called adrenergic receptor agonist) are octopamine (ortho-, meta- or para-octopamine, preferably para-octopamine), synephrine (ortho-, meta- or para-synefrine, preferably para-synephrine), norepinephrine, epinephrine, ephedrine, phenylpropanolamine, tyramine, epinine, phenylethanolamine, beta-phenylethylamine, hordenine, isopropylnorsynephrine, N-methyltyramine, salbutamol, levosalbutamol, terbutaline, pirbuterol, procaterol, clenbuterol, metaproterenol, fenoterol, bitolterol, ritodrine, isoprenaline, salmeterol, formoterol, bambuterol, clenbuterol, olodaterol, indacaterol, Amibegron (SR-58611A), CL 316,243, L-742,791, L-796,568, LY-368,842, Mirabegron (YM-178), Ro40-2148, CGP12177, Solabegron (GW-427,353) and BRL 37,344.

Preferred beta agonists are octopamine (ortho-, meta- or para-octopamine, preferably para-octopamine), synephrine (ortho-, meta- or para-synefrine, preferably para-synephrine), norepinephrine, epinephrine, ephedrine, phenylpropanolamine, tyramine, epinine, phenylethanolamine, beta-phenylethylamine, BRL 37,344, hordenine, isopropylnorsynephrine, N-methyltyramine , isoprenaline, Amibegron (SR-58611A), L-742,791, L-796,568, LY-368,842, Mirabegron (YM-178), Ro40-2148, CGP12177 and Solabegron (GW-427,353).

More preferred beta agonists are octopamine (ortho-, meta- or para-octopamine, preferably para-octopamine), synephrine (ortho-, meta- or para-synefrine, preferably para-synephrine), isoprenaline, SR 58611A, CGP12177, even more preferred are (ortho-, meta- or para-octopamine, preferably para-octopamine), synephrine (ortho-, meta- or para-synefrine, preferably para-synephrine) and isoprenaline, more preferred are para-octopamine, para-synephrine and isoprenaline, and most preferred are para-octopamine and para-synephrine, preferably para-octopamine.

Among beta agonists, beta-3 agonists are preferred. Beta-3 agonists are for example octopamine (ortho-, meta- or para-octopamine, preferably para-octopamine), synephrine (ortho-, meta- or para-synefrine, preferably para-synephrine), isopropylnorsynephrine, Amibegron (SR-58611A), L-742,791, L-796,568, LY-368,842, Mirabegron (YM-178), Ro40-2148, Solabegron (GW-427,353), CGP12177 and BRL 37,344.

Most preferred beta-3-agonists are (ortho-, meta- or para) octopamine and (ortho-, meta- or para) synefrine, most preferably para-octopamine and para-synephrine.

Examples of suitable alpha-agonists, in particular alpha-2 agonists, are 4-NEMD, 7-Me-marsanidine, Agmatine, Apraclonidine, Brimonidine, Clonidine, Detomidine, Dexmedetomidine, Fadolmidine, Guanabenz, Guanfacine, Lofexidine, Marsanidine, Medetomidine, Methamphetamine, Mivazerol, Rilmenidine, Romifidine, Talipexole, Tizanidine, Tolonidine, Xylazine, Xylometazoline and TDIQ.

Preferred alpha-agonists are xylometazoline, clonidine, guanabenz, xylazine and guanfacine, and most preferred alpha-agonists are xylometazoline and xylazine, most preferably xylazine.

Antagonists for the adrenergic receptor include but are not limited to beta antagonists, beta-3 antagonists and alpha-2 antagonists. Preferably, beta-3 antagonists are used in the present context. Whether a compound is an antagonist for an adrenergic receptor can be determined as described above.

Examples of suitable beta antagonists are Carteolol, Nadolol, Penbutolol, Pindolol, Propranolol, Sotalol, Timolol, Acebutolol, Atenolol, Betaxolol, Bisoprolol, Celiprolol, Esmolol, Metoprolol, Nebivolol, Bucindolol, Carvedilol and Labetolol, L-748,328, L-748,337 and SR 59230A. Preferred beta antagonists are Penbutolol, Pindolol, Propranolol, Atenolol, Metoprolol L-748,328, L-748,337 and SR 59230A. Preferably, a beta adrenergic receptor antagonist of the invention is propranolol, SR 59230A or metoprolol, most preferably propranolol or SR 59230A, most preferably SR 59230A.

Examples of suitable beta-3 antagonists are L-748,328, L-748,337 and SR 59230A.

Examples of suitable alpha-antagonists, in particular alpha-2 antagonists, are Aripiprazole, Asenapine, Atipamezole, Cirazoline, Clozapine, Efaroxan, Idazoxan, Lurasidone, Melperone, Mianserin, Mirtazapine, Napitane, Olanzapine, Paliperidone, Risperidone, Phenoxybenzamine, Phentolamine, Piribedil, Rauwolscine, Rotigotine, Quetiapine, Norquetiapine, Setiptiline, Tolazoline, Yohimbine, Ziprasidone, Zotepine, preferably Yohimbine.

A prodrug is defined as a compound which comprises an agonist or an antagonist for the adrenergic receptor as defined above, which is covalently bound to a hydrolyzable moiety. In vivo, the prodrug releases the agonist or antagonist by hydrolysis of the covalent bond between agonist or antagonist and hydrolyzable moiety, thereby releasing the prodrug. A prodrug of the invention has an octanol/water partition coefficient (logP) of at least 0, preferably at least 1, more preferably at least 2, more preferably at least 2.3, even more preferably at least 2.5, even more preferably at least 3. Preferably, the hydrolyzable moiety is more hydrophobic than the free agonist or antagonist, so that the prodrug has a higher logP than the free agonist or antagonist.

A hydrolyzable moiety in this context can be called lipophilic. Lipophilic in this context means that its logP is at least 0, preferably at least 1, more preferably at least 2, more preferably at least 2.3, even more preferably at least 2.5, even more preferably at least 3. Generally, such groups are known, and comprise alkyl groups.

Suitable alkyl groups are preferably C2-C32 alkyl groups, preferably C2-C24, more preferably a C4-C20 linear or branched, saturated or unsaturated alkyl group, more preferably a C2-C24, more preferably a C4-C20 linear alkyl group. Even more preferably, the alkyl group is a C5-C18 saturated or unsaturated alkyl group, such as an alkyl group derived form pentanoic acid, heptanoic acid, octanoic acid or decanoic acid, more preferably a C7-C12 saturated or unsaturated fatty alkyl group. Alkyl groups in this context may be branched, but preferably, the alkyl group is linear, with a functional group on one end which is used for attachment to the agonist or antagonist. The linear chain may be saturated or may comprise one or more double bonds. Suitable functional groups used for attachment to the agonist or antagonist are for example carboxylic acid groups, acid halides or isocyanates.

Upon administration of the prodrug to an individual, the prodrug is hydrolyzed in vivo, for instance by the action of endogenous enzymes as defined above, to release the agonist or antagonist.

The prodrug is an ester. A prodrug of the invention, upon absorption into fat tissue, is hydrolyzed in vivo, to release an adrenergic receptor agonist or an adrenergic receptor antagonist, which subsequently modulates subcutaneous fat tissue by agonistic or antagonistic action on the adrenergic receptor.

In a much preferred embodiment, a prodrug of the invention is an ester of a adrenergic receptor agonist. In an alternative preferred embodiment, the prodrug of the invention is an ester of a adrenergic receptor antagonist.

An ester, in this context, is a compound esterified on a free -OH group with an ester group by reaction with an acylating agent. Preferably, the ester group is a lipophilic ester group. Preferably, the free -OH group is located on a phenyl ring of the adrenergic receptor agonist or antagonist. Further preferably, all free -OH groups are substituted with a lipophilic ester group.

An ester group in this context can be lipophilic. Lipophilic in this context means that its logP is at least 0, preferably at least 1, more preferably at least 2, more preferably at least 2.3, even more preferably at least 2.5, even more preferably at least 3. Generally, such groups are known, and comprise hydrophobic ester groups such as for example alkyl esters, such as preferably C2-C32 alkyl esters.

Preferably, the ester group is a C2-C24, more preferably a C4-C20 linear or branched, saturated or unsaturated alkyl ester, more preferably a C2-C24, more preferably a C4-C20 fatty acid ester. Even more preferably, the ester group is a C5-C18 saturated or unsaturated fatty acid ester, such as a pentanoate, heptanoate, octanoate or decanoate ester, more preferably a C7-C12 saturated or unsaturated fatty acid ester, and most preferably a decanoate ester. A fatty acid in this context may be branched, but preferably, the fatty acid is linear. Preferably, a fatty acid in this context is a naturally occurring fatty acid, i.e. a linear chain of *n* carbon atoms with a carboxylic acid group on one end, which linear chain may be saturated or may comprise one or more double bonds.

For high skin penetration, an alternative preferred fatty acid ester group is a pentanoate ester, a heptanoate ester, an octanoate ester or a decanoate ester, most preferably a pentanoate ester.

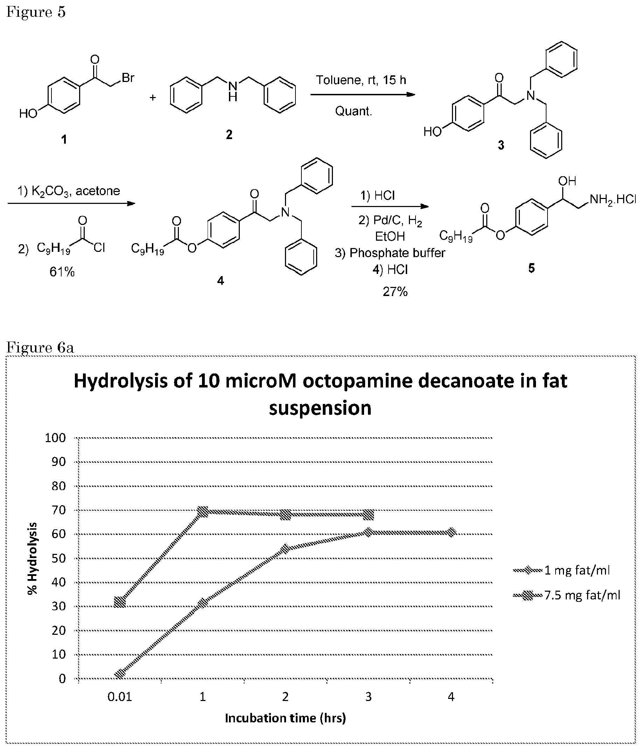

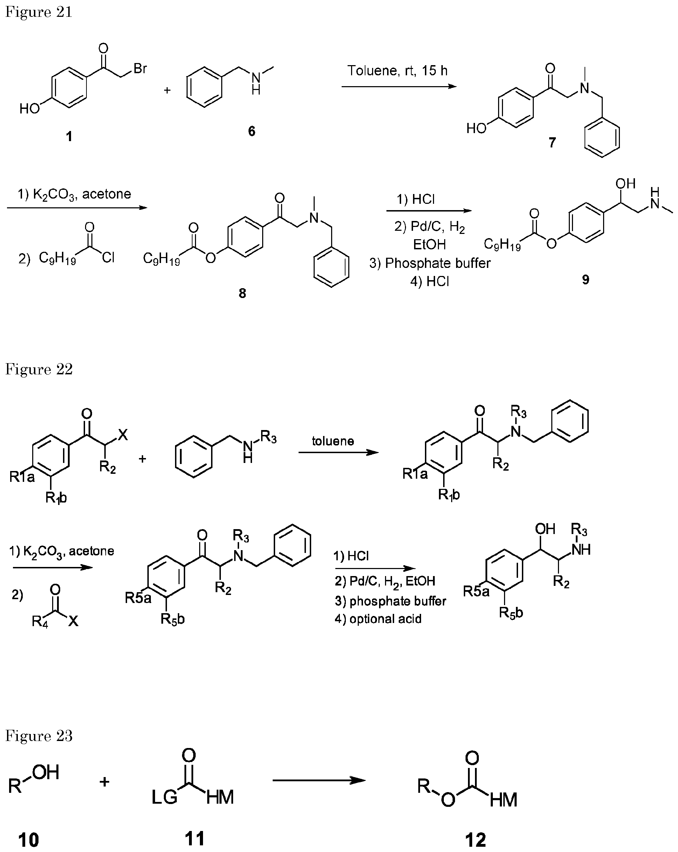

Prodrugs can be prepared by according to many synthetic routes. Someone skilled in the art of organic chemistry can come up with countless ways of synthesizing a prodrug of the invention. A suitable route toward decanoate ester prodrugs is depicted in Figure 5. This route may be adapted to obtain other alkyl ester prodrugs of the invention, by replacing the decanoyl chloride with a different acylating agent. An example of this is the use of valeroyl chloride instead of decanoyl chloride (C₉H₁₉COCl) to obtain octopamine pentanoate. In addition, a different amine moiety may be obtained by substuting dibenzylamine 2 with a different amine. An Example of this is depicted in Figure 21, where benzylmethylamine 6 is used instead of dibenzylamine 2. The method may also be readily adapted to substitute the phenol core derived from 1 with a another phenol or bisphenol, and appropriate modification of the quantity of reagents used.

Thus, a potential general synthetic route toward prodrugs is depicted in figure 22, wherein R₁ₐ and R_{1b} is H or OH, and at least one of R₁ₐ and R_{1b} is OH, R₂ is H or methyl, X is a leaving group, preferably chloride, bromide or iodide, R₃ is benzyl or alkyl (preferably methyl or isopropyl), R₄ is a C1-C31 alkyl group to provide the C2-C32 alkyl ester as defined above, and wherein at least of R₅ₐ and R_{5b} is R₄CO.

In general, an ester prodrug of the invention can also be made by esterifying an adrenergic receptor agonist or an adrenergic receptor antagonist with a hydrolyzing moiety functionalized to result in ester formation. This can be an acylating agent; the acylating agent is selected such that esterification results in the substitution of the free -OH group with a suitable ester group. Esterification is suitably achieved in solution, preferably at a temperature of 0-140 °C, more preferably 20 - 100 °C, preferably under acidic or basic conditions as is known in the art. Basic conditions are preferred. Preferably, the prodrug is subsequently isolated and/or purified. Suitable methods of isolation and/or purification include for example extraction, chromatography and crystallization, and are well known in the art.

Suitable acylating agents are for example acid halides, preferably acid chlorides, of C4-C20 linear or branched, saturated or unsaturated acids, or anhydrides of C4-C20 linear or branched, saturated or unsaturated alkyl acids. Examples of suitable acylating agents are heptanoyl chloride, octanoyl chloride, decanoyl chloride, dodecanoyl chloride, heptanoic anhydride, octanoic anhydride, decanoic anhydride and dodecanoic anhydride. However, the skilled person can come up with countless ways to achieve esterification using various acylating agents, and these are not to be excluded.

A alternative generalized scheme for formation of an ester prodrug 12 is for example depicted in Figure 23, wherein 10 is an agonist or antagonist for an adrenergic receptor as described above, which has a free OH-group, which free OH-group is preferably a benzylic or phenolic OH- group; and 11 is an acylating agent, preferably an acid halide or an anhydride, wherein LG is a leaving group, preferably selected from a halide (preferably chloride), or a carboxylate, and wherein HM is a hydrolyzable moiety as defined above. Conditions for performing such reactions are well-known in the art.

Suitable solvents for esterification are known in the art, and include preferably polar aprotic solvents such as DMF, DMSO, and pyridine, polar protic solvents such as methanol, ethanol, or aromatic solvents such as toluene or xylene.

Suitable acids to achieve acidic conditions during esterification are known in the art, and include for instance HCl, H₂SO₄, HNO₃ or acetic acid. Suitable bases to achieve basic conditions during esterification are also known in the art, and include for instance KOH, NaOH, LiOH or pyridine.,

If the prodrug is an amide, the amide group is preferably formed on a free N-H group of the adrenergic receptor agonist or antagonist. Methods for making amide prodrug can readily be devised by the skilled person. For example, a potential method is to convert a free amine form of the prodrug to an amide by assisted coupling of the free amine to a hydrolyzable moiety through a carboxylic acid as functional group. Suitable agents for assisted coupling, and how to use them, are well known, and include for instance DCC, EDCI, HATU or HBTU. Alternatively, the agonist or antagonist may be reacted with an acylating agent as described above. Preferably, free phenolic and benzylic -OH groups are protected during this reaction by a suitable protective group, such as for example a methoxymethyl ether (MOM) group.

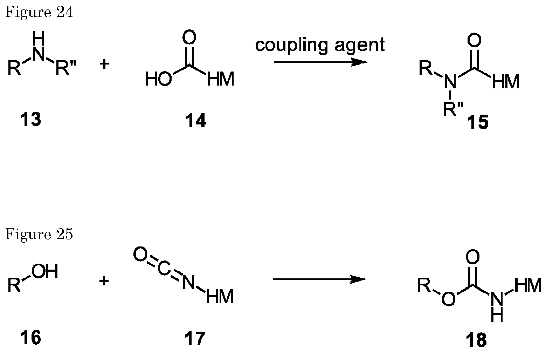

A general route for formation of an amide prodrug 15 is for example depicted in Figure 24, wherein 13 is an agonist or antagonist for an adrenergic receptor as described above, which has a free amine group comprising at least one amine hydrogen, which free amine group is preferably a primary alkyl amine, wherein R" is selected from H or a C1-C8 linear, branched or cyclic alkyl group such as methyl, ethyl or isopropyl, preferably H, and wherein preferably, free OH-groups, more preferably phenolic or benzylic free OH-groups, are protected by a suitable protecting group; and wherein 14 is a hydrolyzable moiety as defined above functionalized with a carboxylic acid group, and wherein the coupling agent can be any known coupling agent, such as for instance DCC, EDCI, HATU or HBTU. Conditions for performing such reactions are well-known in the art.

Carbamate prodrugs can be prepared for instance by reaction of a free OH-group of the agonist or antagonist, preferably a phenolic -OH group, with an hydrolyzable moiety comprising an isocyanate functional group. A general route for formation of a carbamate prodrug 18 is for example depicted in Figure 25, wherein 16 is an agonist or antagonist for an adrenergic receptor as described above, which has a free OH-group, which free OH-group is preferably a benzylic or phenolic OH- group; and wherein 17 is a hydrolyzable moiety as defined above, functionalized with an isocyanate. Conditions for performing such reactions are well-known in the art.

The invention thus also relates to a prodrug for an agonist and/or an antagonist for an adrenergic receptor, wherein the prodrug has an octanol/water partition coefficient of at least 0, as further defined above. The prodrug may be any prodrug defined above. The invention further pertains to a method of making a prodrug of an adrenergic receptor agonist or antagonist, comprising coupling an adrenergic receptor agonist or an adrenergic receptor antagonist with a suitably functionalized hydrolyzable moiety, and isolating the prodrug. Optionally, the prodrug is subsequently purified, such as by chromatography or crystallization. Preferably, the coupling is achieved through esterification with an acylating agent.

The modulation of subcutaneous fat tissue by the composition of the invention comprises decreasing the quantity of subcutaneous fat (adipose) tissue, increasing the quantity of subcutaneous fat tissue, or reinforcing subcutaneous fat tissue. This is achieved by the affinity of the prodrug for fat tissue, which results in enhanced absorption of the prodrug in the fat tissue, so that after release of the agonist or antagonist from the prodrug by the action of endogenous enzymes, subcutaneous fat tissue responds to an increased presence of the agonist or antagonist.

The affinity of the prodrug for fat tissue means that the prodrug is almost fully absorbed into the fat tissue. The prodrug is less prone to be absorbed into blood, and preferably partitions into fat tissue. This avoids fast hydrolysis of the prodrug in blood, and therefore high systemic concentration of the agonist and/or antagonist, precluding the side effects associated with increased plasma presence of adrenergic receptor agonists or antagonists. Therefore, the local concentration of prodrug in the subcutaneous fat tissue results in a local increased concentration of the agonist or antagonist, without significantly affecting the systemic concentration of the agonist or antagonist.

Subcutaneous fat tissue, in the present context, is a tissue layer just beneath the skin of a mammal, comprising adipocytes. It is a tissue layer in which fat is stored as triglycerides.

Too much stored fat in subcutaneous fat tissue may appear as excess body volume, which can be reduced by modulation of the fat tissue as herein described. Such excess fat is often experienced at the abdomen, hips, buttocks or upper legs, and these locations are sometimes indicated as renowned "problem areas" in weight loss or body sculpting programs. Consequently, these locations are preferred sites for topical administration of compositions of the invention, in particular for compositions comprising a prodrug for an agonist of a beta adrenergic receptor and/or a prodrug for an antagonist of an alpha adrenergic receptor. Such compositions have the effect of decreasing the quantity of subcutaneous fat tissue. The decrease in the quantity of fat tissue can be measured by measuring BMI, waist-, hips-and breast circumference or skinfold.

Too little stored fat in the fat tissue may appear as overly slim. This can be modulated in accordance with the invention by increasing the amount of subcutaneous fat tissue as herein described ("plumping"). Overly slim locations are often experienced at the face, breast, buttocks or hips parts of the body, and these locations are preferred sites for topical administration of compositions of the invention, in particular for compositions comprising a prodrug for an antagonist for a beta adrenergic receptor, and/or a prodrug for an agonist of agonist of an alpha adrenergic receptor. This has the effect of increasing the quantity of subcutaneous fat tissue.

Alternatively, body fat may be present in an uneven layer. In this case, sections with relatively little body fat may be treated with a composition for topical administration of the invention which increases the quantity of subcutaneous fat tissue, so as to achieve a more even layer of fat tissue. It is particularly advantageous in this context to apply two compositions according to the invention, at different locations: one treatment which decreases subcutaneous fat tissue at locations where there is excess fat, and one treatment which increases subcutaneous fat tissue at locations where there is relatively little fat. This way, it is possible to achieve a layer of subcutaneous fat tissue of more or less even thickness.

The increase in the quantity of fat tissue can be measured by measuring BMI, waste-, hips- and breast circumference or skinfold.

Also, subcutaneous fat may loose its internal structure or strength, which may lead to formation of wrinkles or cellulite ("orange peel syndrome"). This can be countered by application of compositions according to the invention which reinforce the subcutaneous fat tissue. Loss of internal structure or strength can be experienced for example in the face and neck of an individual. These locations are therefore preferred sites of for topical administration of compositions of the invention which reinforce subcutaneous fat tissue. Alternatively, cellulite usually occurs on the upper legs, thighs and buttocks of an individual, and these are therefore also preferred sites of application of compositions according to the invention which reinforce subcutaneous fat tissue. Reinforcing the fat tissue means that fat tissue is affected by the compounds and method of the invention by increasing strength.

This can be determined by for instance with a profilometric method for measuring the size and function of the wrinkles. Wrinkle size can be measured in relaxed conditions and the representative parameters are considered to be the mean 'Wrinkle Depth', the mean 'Wrinkle Area', the mean 'Wrinkle Volume', and the mean 'Wrinkle Tissue Reservoir Volume' (WTRV). Severity of cellulite can be determined by visual inspection or wrinkle depth.

Reinforcement of subcutaneous fat tissue can be achieved by a prodrug for an agonist and/or for an antagonist. Preferably, this is achieved by a composition comprising one or more prodrugs for one or more of an agonist and an antagonist for an adrenergic receptor, so as to achieve inhibition and/or activation of beta and alpha adrenergic receptors.

Administration of a composition according to the invention is furthermore effective in removing cellulite, either by decreasing the quantity of subcutaneous fat tissue, or by reinforcing said tissue. Thus the invention further pertains to a prodrug for an adrenergic receptor agonist or an adrenergic receptor antagonist as elsewhere described, for use in a method for removing cellulite, and to compositions comprising this prodrug. Preferably, the prodrug is a beta agonist or an alpha antagonist, more preferably a beta agonist, more preferably a beta-3 agonist, most preferably octopamine, synephrine or isoprenaline, preferably octopamine. Most preferred prodrugs are prodrugs based on a C5-C12 hydrolyzable moiety, such as, preferably pentanoate, heptanoate, octanoate, or decanoate ester prodrugs.

Generally, shaping a mammalian body by modulation of subcutaneous fat tissue results in increased or decreased volume of the subcutaneous fat tissue, or reinforcing subcutaneous fat tissue. Preferably, the effect of shaping is a local effect, which means the effect occurs at the site of application, and not at sites where the prodrug has not been applied. In this embodiment, the prodrug targets the subcutaneous fat tissue locally, at the site of application, to achieve local modulation of subcutaneous fat tissue.

If the composition comprises a prodrug for a beta adrenergic receptor agonist or an alpha adrenergic receptor antagonist, the modulation preferably comprises decreasing the quantity of subcutaneous fat tissue. This results in hydrolysis of the fat triglycerides and in liberation of fatty acids and glycerol into blood.

If the composition comprises a prodrug for a beta adrenergic receptor antagonist or an alpha adrenergic receptor agonist, the modulation preferably comprises increasing the quantity of subcutaneous fat tissue.

It is an advantage of the present invention that sites on a mammalian body which require an increase or decrease in fat tissue volume can be targeted by the agonist or antagonist at will, while avoiding high systemic concentrations of the agonist(s) and/or antagonist(s).

It is a further advantage of a prodrug of the invention that its logP is at least 0, as elsewhere described. This increases the transdermal absorption through the skin, relative to agonists or antagonists themselves, thus increasing the rate of absorption and also the quantity of prodrug that can be absorbed, which allows increased modulation of subcutaneous fat tissue. Penetration is preferably enhanced by the presence of a lipophilic group, such as a hydrolyzable moiety as defined above. In addition, the prodrug with a logP as defined preferably partitions in fat tissue, which further enhances the modulation of fat tissue by the agonist or antagonist after release from the prodrug. The prodrug of the invention has inclusion of a lipophilic ester group on the agonist or antagonist for a adrenergic receptor

It is a specific advantage of the prodrugs for beta adrenergic receptor agonists and/or alpha adrenergic receptor antagonists, that their mode of action is synergistic with the achieved effect. Upon hydrolysis of the prodrug in subcutaneous fat tissue, the beta agonist and/or alpha antagonist stimulate the action of lipase. Lipase not only hydrolyses triglycerides to result in the decrease in subcutaneous fat tissue, but also hydrolyses the prodrug itself. Thus, administration of the prodrug results not only in increased lipase action, but also in increased hydrolysis of the prodrug. This provides for a self-reinforcing (auto-catalytic) effect in the action of the prodrug on triglyceride hydrolysis, as the product of the hydrolysis (the agonist or antagonist), stimulates even further increased action of lipase. This cycle makes this embodiment particularly effective in decreasing the quantity of subcutaneous fat tissue.

If the composition comprises, apart from a prodrug for an agonist for a beta adrenergic receptor or an antagonist for an alpha adrenergic receptor, also a phosphodiesterase inhibitor, the effect on beta receptor stimulation or alpha receptor inhibition is amplified. Thus, in a particularly preferred embodiment, the composition comprises a prodrug for a beta agonist and/or an alpha antagonist as well as a phosphodiesterase inhibitor, or a prodrug thereof, or an adenyl cyclase stimulator or a prodrug thereof, to enhance the lipolytic effect. Suitable adenyl cyclase stimulators or phosphodiesterase inhibitors are forskolin, caffeine and theophylline, preferably caffeine. A prodrug for a phosphodiesterase inhibitor or an adenyl cyclase stimulator is defined in line with a prodrug for and adrenergic receptor agonist or - antagonist.

A prodrug of the invention may be optimized for transdermal application by variation of the lipophilic group. A preferred prodrug of the invention is a lipophilic ester of an adrenergic receptor agonist, in particular to decrease the quantity of subcutaneous fat. An alternative preferred prodrug is a lipophilic ester of an adrenergic receptor antagonist, in particular to increase the quantity of subcutaneous fat. Lipophilic is defined as having an affinity for apolar environments rather than polar environments, and a lipophilic group is a group which increases the affinity for apolar environments. Much preferred as a prodrug in the present composition is a lipophilic ester of an adrenergic receptor agonist, preferably a beta-3 adrenergic receptor agonist.

Prodrugs of agonists or antagonists in accordance with the invention may be neutral molecules, but may also have any pharmaceutically acceptable salt-form, or be complexed to a pharmaceutically acceptable molecule, for instance as a stabilizer. Suitable salts are for instance bromides, chlorides, tartrates, or citrates.

Also, prodrugs of the invention which have one or more stereocenters may have an *R* or an *S* configuration on either stereocenter, or be mixtures of *R* and *S* stereoisomers. Thus, prodrugs of the invention may be pure stereoisomers or a stereoisomeric mixture of asy proportion, including enantiomeric mixtures and diastereomeric mixtures. Preferably, the prodrugs are a racemic mixture.

The invention further relates to pharmaceutical compositions for topical administration comprising one or more agonists for an adrenergic receptor, one or more antagonists for an adrenergic receptor, as well as one or more prodrugs for an agonist and/or antagonist for an adrenergic receptor. As such, the composition comprising one or more prodrugs may also comprise one or more "free" agonists and/or antagonists. Free, in this context, means that the agonist or antagonist is not covalently bound to a hydrolyzable moiety, but instead is in the form in which it has most affinity for the adrenergic receptor. Preferably in this embodiment, free agonists and antagonists also have a log P of at least 0. More preferably, free agonists and antagonists have a log P of at least 1, more preferably at least 2, more preferably at least 2.3, more preferably at least 2.5, and most preferably they have a log P of at least 3.

The free agonist and/or antagonist may have the same or opposite effect on lipase action as the prodrug. It is preferred if the agonist and/or antagonist has the same action on lipase action as the prodrug. A composition comprising one or more prodrugs for a beta agonist and/or an alpha antagonist may therefore also comprise one or more free beta agonists and/or free alpha antagonists. Also preferred is a composition comprising one or more prodrugs for a beta antagonist and/or one or more prodrugs for an alpha agonist which also comprises one or more free beta antagonists and/or one or more free alpha agonists.

In a preferred embodiment, the composition comprises , as mol % of total agonists, antagonists and prodrugs, at least 5 %, preferably at least 10 %, more preferably at least 20 %, more preferably at least 30 %, more preferably at least 40 %, more preferably at least 50 %, more preferably at least 60 %, more preferably at least 70 %, more preferably at least 80 %, more preferably at least 90 %, more preferably at least 95 %, and most preferably at least 98 % prodrug.

The invention preferably also pertains to compositions comprising a combination of one or more prodrugs for one or more adrenergic receptor agonists and one or more prodrugs for one or more adrenergic receptor antagonists, which may further comprise free agonists and/or antagonists as described above. Preferably, the composition comprises one or more prodrugs for one or more beta adrenergic receptor agonists and/or one or more prodrugs for one or more alpha adrenergic antagonists. An alternative preferred composition comprises one or more prodrugs for one or more beta adrenergic receptor antagonists and/or one or more prodrugs for one or more alpha adrenergic receptor agonists.

In a pharmaceutical composition of the invention, the prodrug is preferably present at a concentration of 0.001 - 1000 mg/ml, more preferably 0.01-100 mg/ml, even more preferably 0.1-10 mg/ml. The density of the hydrogel may be between 0.8 and 1.2 g/ml, preferably between 0.9 and 1.1 g/ml. Free agonists or antagonists, if present, may be present in the same concentration ranges.

A composition of the invention preferably also comprises a pharmaceutically acceptable carrier. Suitable carriers include, but are not limited to a hydrogel, cutaneous cream, cutaneous shampoo, cutaneous foam, cutaneous powder, cutaneous gel, cutaneous lotion, cutaneous ointment, cutaneous patch, medicated plater, cutaneous paste, cutaneous poultices, cutaneous solution, cutaneous spray, iontoforesis patch, sticks or injectables. A preferred carrier is a hydrogel, a cutaneous cream, cutaneous gel, cutaneous lotion, or cutaneous ointment.

Further preferably, the pharmaceutical composition is a cream, foam, gel, lotion, ointment, patch, paste, solution or spray, preferably a cutaneous gel or a cutaneous lotion. Alternatively, the pharmaceutical composition is pharmaceutically acceptable for microinjection or iontoforesis.

The prodrug of the invention is preferably applied to the skin at 0.001-1000 mg/cm², preferably 0.01-100 mg/cm², most preferably 0.1-10 mg/cm². Free agonists or antagonists, if present, may be applied in the same concentration ranges.

The composition may additionally comprise one or more pharmaceutically acceptable excipients. Suitable excipients are transdermal absorption improvers, stabilizers, colorants, emulsifiers preservatives, viscosity enhancers, humectans, odor improvers and skin tighteners.

Examples of suitable transdermal absorption improvers are niacin esters, organic solvents (dimethylsulfoxide, alcohols and alkanols (ethanol, decanol), propylene glycol, azones, pyrrolidones, terpenes), surfactants (detergents), ureum and salicylic acid.

Examples of suitable stabilizers are antioxidants. Suitable antoixoidants are known in the art, and may be water-soluble or fat-soluble. Suitable fat-soluble stabilizers and antioxidants are dl-alpha-tocopherol and butylhydroxytoluene; suitbale water-soluble stabuilizers and antooxidants are ascorbic acid, sodium pyrosulfite and disodium edetate.

Examples of suitable colorants are iron oxides (yellow, red, brown), and chlorophyll.

Emulsifiers can be ionogenic or non-ionogenic. Examples of ionogenic emulsifiers are alkyl sulfates and quaternary ammonium salts. Examples of non-ionogenic emulsifiers are polyethylene glycol, fat, alcohol ethers (cetomacrogols), sorbitan olest (=Span 80) and polyethylene glycol sorbitan ethers (= polysorbate 80, Tween 80).

Examples of suitable preservative are parabens (parahydroxybenzoic acid derivatives), sorbic acid, chlorhexidine digluconate, cetrimide, phenol, phenoxyethanol, propylene glycol, ethanol and glycerol.

Examples of viscosity enhancers are cellulose derivatives, inorganic colloids, polyacrylic acid derivates (carbomers) and natural viscosity enhancers, such as for example tragacanth.

Examples of humectans are glycerol, polyethylene glycol and sorbitol 70%.

Examples of odor improvers are rose oil, lavender oil, and other essential oils.

Examples of skin tighteners are collagen, PhytoCellTec stem cells, growth factors, peptides, tripeptides, hexapeptides, antioxidants, emollients, sebum-controllers, anti-inflammatories, collagen producers, phytosterols, glycolipids and polyphenols

The invention further relates to a cosmetic method of shaping a mammalian body by locally modulating subcutaneous fat tissue, comprising topically administering a pharmaceutical composition as defined above.

In a cosmetic method of the invention, a mammalian body can be shaped by modulating subcutaneous fat tissue locally. In cosmetic methods of the invention, decreasing or increasing the quantity of fat tissue changes the physical appearance of a subject. This effect allows a subject to increase or decrease the volume of subcutaneous fat tissue at will at any site of the body where such effect is wanted. As subcutaneous fat is often associated with the "problem areas" which individuals actively pursuing an attractive physical appearance consider particularly difficult to address, this allows for targeted local modulation of those areas. Thus, a more attractive physical appearance can be attained, without therapeutic benefit. Particularly preferred for this purpose are prodrugs for agonists for the beta adrenergic receptor, preferably the beta-3 receptor.

In addition, cosmetic methods of the invention allow the treatment of wrinkles, by decreasing or increasing the quantity of subcutaneous fat tissue, or by reinforcing the subcutaneous fat tissue.

Alternatively, the compositions of the invention can be used in a medical treatment to attain a healthy body weight in a mammal, such as in a treatment to obtain a healthy weight for over- or underweight individuals by modulating subcutaneous fat tissue. Particularly preferred is a prodrug for a beta adrenergic receptor agonist, preferably a beta-3 adrenergic receptor, , for use in a method of attaining healthy body weight in overweight individuals by decreasing subcutaneous fat tissue. This can be highly advantageous in the treatment or prevention of obesitas and type II diabetes.

The cosmetic method and the medical treatment can be distinguished by the patient type. In individuals with an increased risk of obesitas or diabetes type II caused by overweight, evaluated by a BMI above 25, administration of a composition for decreasing subcutaneous fat tissue according to the invention is medical. In individuals who do not have an increased risk, such as in individuals having a BMI below 25, administration of a composition for decreasing subcutaneous fat tissue according to the invention is cosmetic.

As for compositions increasing subcutaneous fat tissue according to the invention, administration to individuals with severe underweight, such as individuals with a BMI below 18.5, is medical, whereas administration of such compositions to individuals with a BMI above 18.5 is cosmetic.

Application of compositions according to the invention which modulate subcutaneous fat tissue by reinforcing said tissue is always cosmetic, independent of patient type.

Compositions according to the invention may be administered as a sole cosmetic or therapeutic treatment as described above, but they may also be combined with further, known treatments. For instance, topical administration of prodrugs according to the invention may be combined with orally administered drugs. Prodrugs of the invention which have the effect of decreasing subcutaneous fat tissue may suitably be combined with oral treatments with the same aim. Such treatments can be for example topical administration of a composition comprising a prodrug for a beta-3 receptor agonist, in combination with oral administration of caffeine.

Alternatively, the invention pertains to use of one or more prodrugs as defined above for the manufacture of a medicament for use in the treatment of obesitas or type II diabetes.

Due to the many variations possible, not all combinations of parameters or groups of parameters can be described. Therefore, any range or possibility described for a particular feature is envisioned to be used with any other range or possibility of another feature.

Clauses which describe pharmaceutical compositions:
1. A pharmaceutical composition for topical administration, comprising an agonist and/or an antagonist for an adrenergic receptor, and/or a prodrug for said agonist or antagonist, wherein the agonist, antagonist or prodrug has an octanol/water partition coefficient of at least 0, for use in a method of shaping a mammalian body by modulation of subcutaneous fat tissue.
2. A pharmaceutical composition according to clause 1, wherein the agonist, antagonist or prodrug has an affinity for subcutaneous fat tissue to achieve local modulation of subcutaneous fat tissue.
3. A pharmaceutical composition according to clause 1 or 2, wherein modulation comprises decreasing or increasing the quantity of subcutaneous fat tissue, or reinforcing the subcutaneous fat tissue.
4. A pharmaceutical composition according to any of clauses 1-3, wherein the agonist is a beta-agonist or an alpha-agonist, and/or wherein the antagonist is a beta-antagonist or an alpha-antagonist.
5. A pharmaceutical composition according to clause 4, wherein
   - the beta-agonist is octopamine, p-synephrine, m-synephrine (phenylephrine), norepinephrine, epinephrine, ephedrine, phenylpropanolamine, tyramine, epinine, phenylethanolamine, beta-phenylethylamine, hordenine, isopropylnorsynephrine, N-methyltyramine, salbutamol, levosalbutamol, terbutaline, pirbuterol, procaterol, clenbuterol, metaproterenol, fenoterol, bitolterol, ritodrine, isoprenaline, salmeterol, formoterol, bambuterol, clenbuterol, olodaterol, indacaterol, Amibegron (SR-58611A), CL 316,243, L-742,791, L-796,568, LY-368,842, Mirabegron (YM-178), Ro40-2148, Solabegron (GW-427,353), BRL 37,344;
   - the alpha-agonist is 4-NEMD, 7-Me-marsanidine, Agmatine, Apraclonidine, Brimonidine, Clonidine, Detomidine, Dexmedetomidine, Fadolmidine, Guanabenz, Guanfacine, Lofexidine, Marsanidine,Medetomidine, Methamphetamine, Mivazerol, Rilmenidine, Romifidine, Talipexole, Tizanidine, Tolonidine, Xylazine, Xylometazoline, TDIQ;
   - the beta-antagonist is Carteolol, Nadolol, Penbutolol, Pindolol, Propranolol, Sotalol, Timolol, Acebutolol, Atenolol, Betaxolol, Bisoprolol, Celiprolol, Esmolol, Metoprolol, Nebivolol, Bucindolol, Carvedilol, Labetolol, preferably Penbutolol, Pindolol, Propranolol, Atenolol, Metoprolol L-748,328, L-748,337, SR 59230A; or
   - the alpha antagonist is Aripiprazole, Asenapine, Atipamezole, Cirazoline, Clozapine, Efaroxan, Idazoxan, Lurasidone, Melperone, Mianserin, Mirtazapine, Napitane, Olanzapine, Paliperidone, Risperidone, Phenoxybenzamine, Phentolamine, Piribedil, Rauwolscine, Risperidone, Rotigotine, Quetiapine, Norquetiapine, Setiptiline, Tolazoline, Yohimbine, Ziprasidone, Zotepine.
6. A pharmaceutical composition according to any of clauses 1 - 5, wherein the prodrug is a lipophilic ester.
7. A pharmaceutical composition according to clause 6, wherein the lipophilic ester is a C4-C20 alkyl ester.
8. A pharmaceutical composition according to clause 7, wherein the lipophilic ester is a butanoate, heptanoate or decanoate ester.
9. A pharmaceutical composition according to any of clauses 1 - 8, wherein the agonist, antagonist or prodrug is present in the composition at a concentration of 0.001-1000 mg/ml.
10. A pharmaceutical composition according to any of clauses 1 - 9, wherein the pharmaceutical composition is a cream, foam, gel, lotion, ointment, patch, paste, solution or spray.
11. A method of shaping a mammalian body by locally modulating subcutaneous fat tissue, comprising topically administering a pharmaceutical composition according to any of clauses 1 - 10.
12. A method according to clause 11, wherein the method is a cosmetic method.
13. A method according to clause 11 or 12, wherein the agonist, antagonist or prodrug is administered at a dosage of 0.001-1000 mg/cm².
14. A lipophilic ester of an adrenergic receptor agonist or an adrenergic receptor antagonist.
15. A lipophilic ester according to clause 14 for medical use, preferably the treatment of type II diabetes or obesity.
16. A lipophilic ester as defined in clause 14 for use in a method of shaping a mammalian body.
17. A method of making a lipophilic ester of an adrenergic receptor agonist or a lipophilic ester of an adrenergic receptor antagonist, comprising esterifying the adrenergic receptor agonist or the adrenergic receptor antagonist with an acylating agent.

The invention will now be illustrated by the following, non-restricting Examples:

### Materials and methods

### Chemicals

Racemic p-Octopamine HCl ("octopamine" or "oct"), racemic p-synephrine ("synephrine" or "syn") and (S)-(-)-Propranolol HCl ("propranolol" or "prop) were obtained at Sigma. p-Octopamine decanoate HCl, p- octopamine pentanoate HCl and p- synephrine decanoate HCl were synthesized by Syncom (Groningen, the Netherlands). L-(-)-Norepinephrine bitartrate was purchased at Sigma Aldrich. Other compounds were regularly obtained from commercial suppliers.

### Carbomer hydrogel

The carbomer hydrogel of examples 3, 4 and 5 was made by dissolving 0.1 g disodium-EDTA (Fagron) in about 50 ml of water. 10 g of propyleneglycol was added. 1 g of carbomer 974P (Fagron) was dispersed using a thurax. Trometamol (Fagron) was dissolved in 10 ml water and added to the carbomer gel. 20 ml of ethanol (96%), with or without octapamine decanoate; see below) was added and thuraxed. Water was added until the total weight was 100g. After homogenization with the thurax, the hydrogel was transferred to 50 ml tubes.

Octopamine decanoate (0.5 g) was maximally dissolved in 20 ml of ethanol (96%). After 5 min sonification, a microsuspension was formed. 20 ml of microsuspension was added to the carbomer hydrogel instead of 20 ml of ethanol. The end concentration of the hydrogel was 5 mg/ml.

(Carbomer) hydrogels in other examples were prepared similarly. The hydrogel was made by dissolving 100 mg disodium EDTA in 50 ml of ultrapure water. 10 gram of propyleneglycol was added and mixed. With a rotor-stator 1 gram of carbomer 974P was dispersed in the solution. 1 gram of trometamol was dissolved in 10 ml of water and mixed with the dispersed carbomer. Water was added to a weight of 80 gram. The hydrogel was stored at 4 °C for use.

In 15 ml 70 % ethanol appropriate quantities of octopamine, octopamine decanoate, octopamine pentanoate and synephrine decanoate, or other prodrugs, were dissolved using sonification at 30 °C for 5 minutes. 15 ml of the obtained ethanolic solution was added to 35 gram of hydrogel and mixed using a rotor stator to attain the indicated final concentrations. Finalized hydrogels were transferred to 50 ml tubes and stored at 4 °C.

Finalized hydrogels comprised 0.73 mmol per 50 ml of hydrogel of free octopamine or octopamine prodrug, or 0.70 mmol per 50 ml of hydrogel of synephrine decanoate. For the making of these hydrogels, 137.8 mg octopamine HCl, 250 mg octopamine decanoate HCl, 199 mg octopamine pentanoate HCl or 250 mg of synephrine decanoate HCl was used.

For hydrogels with a different concentration, the quantities of prodrug can easily be varied to attain a specific concentration of prodrug in the hydrogel.

Hydrogels made according to these preparation procedures have a density of 1.0 g/ml.

### Synthesis of octopamine decanoate prodrug (Figure 5)

### 2-(dibenzylamino)-1-(4-hydroxyphenyl)ethanone (3).

A mixture of bromide **1** (4.8 g, 22.3 mmol) in 35 mL toluene was cooled in ice. Dibenzylamine **2** (8.8g, 8.6 mL, 2 eq.) was added drop wise. The mixture was stirred at RT overnight. The mixture was filtered and the solvent was evaporated to give crude 2-(dibenzylamino)-1-(4-hydroxyphenyl)ethanone (**3**; 9.7 g, quant.) as red solid.

### 4-(2-(dibenzylamino)acetyl)phenyl decanoate (4).

To a mixture of 2-(dibenzylamino)-1-(4-hydroxyphenyl)ethanone **3** (7.35 g, crude, 17.8 mmol) in 100 mL acetone was added K₂CO₃ (4.5 g, 1.8 eq.). The mixture was refluxed for 1 hour. The mixture was cooled and the solid was filtered off. The solvent was evaporated to give 7 g of a yellow oil. This oil was dissolved in 50 mL acetone. To this mixture was added decanoyl chloride (3.1 mL, 1.5 eq.) and Et₃N (3.6 mL, 1.5 eq.). The mixture was stirred at RT overnight. The solid was filtered off and the solvent was evaporated to give 8.4 g yellow oil. This oil was purified by column chromatography (SiO₂, EtOAc/Heptane 1:9) to give 4-(2-(dibenzylamino)acetyl)phenyl decanoate (**4**; 5.25 g, 61%) as yellow oil, which solidified upon standing.

### 4-(2-amino-1-hydroxyethyl)phenyl decanoate (5).

To a mixture of 4-(2-(dibenzylamino)acetyl)phenyl decanoate **4** (5.25 g, 10.8 mmol) in 100 mL ether was added 4N HCl in dioxane (8 mL, 3 eq.). The mixture was stirred at RT for 5 minutes and the solvent was evaporated.

The mixture was dissolved in 100 mL ethanol. To this mixture 500 mg 10% Pd/C was added and the mixture was stirred under 1 bar H₂ for 3 nights. The Pd/C was filtered off and the solvent was evaporated to give 2.5 g of a sticky/foamy solid. This solid was tritured in 7.5 mL phosphate buffer (KH₂PO₄/K₂HPO₃ pH=7) to give a white precipitate. This precipitate was filtered off, washed with water, acetone and dried to give 1.32 g white solid. This material (octapamine decanoate free base, correct?) was taken in 10 mL water. Aqueous HCl was added drop wise until a clear solution. The mixture was lyophilized and the solid was collected to give 4-(2-amino-1-hydroxyethyl)phenyl decanoate (5; 990 mg, 27%) as white waxy solid. Purity (LC-MS): >93%.

### Synthesis of octopamine pentanoate prodrug

The synthesis of octopamine pentanoate was carried out following the same route as depicted in Figure 5, but substituting valeroyl chloride for decanoyl chloride.

### 2-(dibenzylamino)-1-(4-hydroxyphenyl)ethanone (3).

A mixture of bromide **1** (50 g, 232 mmol) in 350 mL toluene was cooled on ice. Dibenzylamine **2** (90 mL, 2 eq.) was added drop wise over 30 minutes. The mixture was stirred at RT overnight. The mixture was filtered and the solvent was evaporated to give crude 2-(dibenzylamino)-1-(4-hydroxyphenyl)ethanone (**3**; 91.6 g, quant.) as a red oil. This oil was used as such in the next step.

### 4-(2-(dibenzylamino)acetyl)phenyl valeroate

To a mixture of 2-(dibenzylamino)-1-(4-hydroxyphenyl)ethanone 3 (30.5 g, crude, assume 77.3 mmol) in 400 mL acetone was added K₂CO₃ (19.5 g, 1.8 eq.). The mixture was refluxed for 1 hour. The mixture was cooled and the solid was filtered off. The solvent was evaporated untill approx. half the volume remained. To this solution was added valeroyl chloride (11.1 mL, 1.2 eq.) and Et₃N (15.6 mL, 1.5 eq.). The mixture was stirred at RT overnight. The solid was filtered off and the solvent was evaporated to give 4-(2-(dibenzylamino)acetyl)phenyl valeroate (**4**; 35.4 g, quant) as yellow oil, which solidified upon standing. This solid was used as such in the next step.

### 4-(2-amino-1-hydroxyethyl)phenyl valeroate hydrochloride (Octopamine pentanoate HCl)

To a mixture of 4-(2-(dibenzylamino)acetyl)phenyl valeroate **4** (12.75 g, crude, around 27 mmol) in 250 mL diethylether was added 4N HCl in dioxane (20 mL, 3 eq.). The mixture was stirred at RT for 10 minutes and the solvent was evaporated. The solid was triturated in diethylether, isolated by filtration and dried. The solid was dissolved in 250 mL ethanol. To this mixture 1 g 10% Pd/C was added and the mixture was stirred under 1 bar H₂ atmosphere for 4 nights. The Pd/C was filtered off and the solvent was evaporated to give 5.8 g of the crude HCl salt as a yellow solid. This solid was triturated in 150 mL phosphate buffer (KH₂PO₄/K₂HPO₃ pH=7) for 1 hour to give a light yellow precipitate. This precipitate was filtered off, washed with water, acetone, diethylether and dried to give 4.7 g of the free base as white solid. This material was taken up in 50 mL water. Aqueous concentrated HCl (2.0 mL, 1.2 eq.) was added drop wise and the mixture was stirred at RT until a clear solution was obtained (around 10 minutes). The mixture was lyophilized and the solid was collected to give 4-(2-amino-1-hydroxyethyl)phenyl valeroate hydrochloride (**5**; 5.1 g, 62% from **1** over 3 steps) as light yellow sticky solid. Purity (LC-MS): >86%.

### Synthesis of synephrine decanoate prodrug

The synthesis of synephrine decanoate was carried out following the same route as depicted in Figure 5, but substituting benzylmethylamine for dibenzylamine. The route is depicted in Figure 21.

### 2-(Benzyl(methyl)amino)-1-(4-hydroxyphenyl)ethan-1-one (7).

To an ice/water cooled suspension of bromide **1** (50.0 g, 233 mmol, 1.0 eq) in toluene (350 mL) was added benzylmethylamine (60.0 mL, 465 mmol, 2.0 eq) and the internal temperature rose from 7 °C to 22 °C. During the reaction, the mixture first became a thin suspension and subsequently a thick suspension formed. The next day, ¹H-NMR showed 92% conversion and additional benzylmethylamine (6.0 mL, 46.5 mmol, 0.2 eq) was added. After another 2 hours of stirring, the reaction mixture was concentrated to dryness. The residue was partioned between water (250 mL) and EtOAc (4 × 400 mL). The combined organic layers were washed with brine (200 mL), dried (Na₂SO₄) and concentrated to dryness. The residue (64.3 g) was taken up in EtOAc and stirred overnight. The resulting suspension was cooled to - 18 °C and the product was collected by filtration (29.9 g). The filtrate was concentrated to dryness and purified by column chromatography (300 g silica, eluted with 1:1 EtOAc:CH₂Cl₂). Pure fractions were pooled and concentrated to dryness and combined with the pure material isolated above. This furnished the title compound as a white solid (50.9 g total, 86% yield).

### 4-(N-Benzyl-N-methylglycyl)phenyl decanoate (8).

Phenol **7** (50.9 g, 199 mol, 1.0 eq) was dissolved in acetone (1000 mL) and treated with K₂CO₃ (49.5 g, 358 mmol, 1.8 eq) and the mixture was heated under reflux for 2 hours. After cooling to room temperature, the solid was removed by filtration. ¹H-NMR of a concentrated sample showed complete conversion of the starting material into the salt.

Decanoyl chloride (18.1 mL, 89.6 mmol, 1.5 eq) was added to 300 mL of the above acetone solution (59.7 mmol potassium salt, 1.0 eq). The resulting thin suspension was treated with Et₃N (12.4 mL, 89.6 mmol, 1.5 eq) and a thick suspension formed while the internal temperature rose to 35 °C. A sample was taken after 3 hours and TLC showed complete consumption of the starting material. The solid was removed by filtration and the filtrate was concentrated to dryness. This furnished a mixture of the title compound and decanoyl chloride (28.5 g, max. 59.7 mmol) which was used as such in the next step.

### 4-(1-hydroxy-2-(methylamino)ethyl)phenyl decanoate (9) (Synephrine decanoate hydrochloride)

To a mixture of 4-(N-benzyl-N-methylglycyl)phenyl decanoate **8** (10.4 g, crude, around 25mmol) in 200 mL diethylether was added 4N HCl in dioxane (15 mL, 3 eq.). The mixture was stirred at RT for 10 minutes and the solvent was evaporated to give a yellow oil. This oil was dissolved in 200 mL ethanol. To this mixture 1 g 10% Pd/C was added and the mixture was stirred under 1 bar H₂ atmosphere for 4 nights. The reaction mixture was filtered through a pad of Celite and then evaporated to dryness. The crude product (10.7 g) was purified by reverse phase column chromatography in batches of 3 grams. The product containing batches were combined. Acetonitrile was removed in *vacuo* and the water layer was removed by freeze drying. The product was obtained as an off-white product (1.9 g, 21%).

### Ethical approval

All experiments were in accordance with ethical guidelines.

### Example 1: Polarity calculations of ester prodrugs

Polarity calculations were made of different esters of the parent compound. Table 1 depicts the results of these calculations. Lipophilicity increased with butanoate, pentanoate, heptanoate and decanoate by generally a logP of 1, 1.5, 2.5 and 4 respectively.

**Table 1. LogP calculations of polarity of ester prodrugs (ACD Chemsketch calculated logP).**

| | Octopamine | Synephrine | Isoprenaline |
|---|---|---|---|
| Freebase | -0.28 | -0.03 | 0.25 |
| Butanoate | 0.86 | 1.11 | 1.04 |
| Pentanoate | 1.39 | 1.64 | 1.57 |
| Heptanoate | 2.45 | 2.70 | 2.63 |
| Decanoate | 4.05 | 4.29 | 4.23 |

### Example 2: In vitro ester hydrolysis assay

Abdominal fat tissue was harvested from male wistar rats (400g, Harlan Zeist) and stored at -80 °C until use. Human fat tissue was obtained from esthetic surgery and stored at -80 °C until use. Tissue was thuraxed (80 mg/ml for rat tissue and 240 mg/ml for human tissue) for 1 min in Phosphate buffered saline (PBS (Irvine Scientific).

Octopamine released was measured using a 50 ml beaker thermostated at 30 degrees. 30 ml PBS (stirred) was pumped at 1 ml/min (Gilson minipulse 3) through a UV detector (SPD-10Avp Shimadzu UV/VIS) set at 280 nm and 2.56 AUFS. The outlet was recirculated back to the beaker. UV signal was recorder on a flatbed recorder (Kipp), set at 1 mm/min and 1 mV gain.

Octopamine and octopamine decanoate solutions (1 mM) were made in ultrapure water adding 1 microliter/ml of 1.8% hydrochloride acid. Octopamine decanoate was sonicated for 30 min at 30 degrees.

Upon stabilization of the UV detector on PBS, 1 ml of tissue suspension was added to the PBS. Upon stabilization of the UV signal, 5 ml of 1 mM of octopamine decanoate was added. Control experiments were performed without addition of tissue suspension in order to monitor spontaneous hydrolysis of decanoate ester in PBS.

### Results

UV spectra were taken from octopamine and octopamine decanoate. Octopamine showed a UV absorption maximum at 279 nm, whereas octopamine decanoate showed a maximum at 269 nm. Relative intensity of octopamine was about 10 times higher than octopamine decanoate.

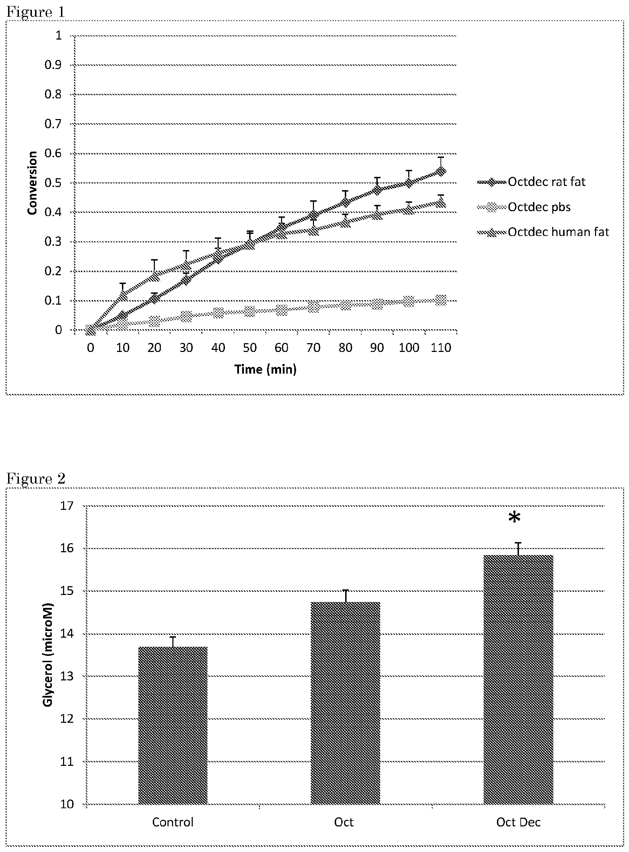

Figure 1 shows the results of the in vitro hydrolysis experiments. Octopamine decanoate spontaneously hydrolyzed into octopamine at a rate of 10% per 110 min. During presence of rat fat suspension, octopamine decanoate hydrolyzed much faster, yielding over 50% of free octopamine within 110 min. In presence of human fat tissue at concentrations three times higher than rat tissue, decanoate hydrolyzed yielding over 40% conversion in 110 min.

From this, it is apparent that hydrolysis of an ester prodrug of octopamine, octopamine decanoate, is faster in the presence of fat tissue than in PBS. This must be caused by the presence of fat tissue, such as for instance endogenous enzymes, among which lipase, which is known to hydrolyze esters.

### Example 3 In vitro assay of effect of octopamine decanoate on glycerol production in human fat.

Human fat tissue was pottered using a teflon potter in 10 ml PBS with 6 mM Glucose (2.4 gram fat tissue per 10ml). The suspension was transferred to a 50 ml beaker and was gently stirred at 37 degrees Celsius to maintain the homogeneous nature. 0.3 ml suspension was transferred to 2 ml reaction vials, spiked with octopamine (end concentration 1 microM) and octopamine decanoate (end concentration 10 microM and PBS/glucose were added until 2ml end volume. Vials were incubated at 37 degrees Celsius for 2 hours.

Samples were spun off at 4000 rpm for 2 min and 10 microliter samples were taken after removal of floating fat with a tissue.

Samples were analyzed using an enzymatic kit (Sigma Aldrich) and fluorescence intensity was measured using direct flow injection (Vici valve in combination with Shimadzu 10 ADvp HPLC pump at 0.15 ml/min, thermo 15*2.1 C18 column prior to valve and using ultrapure water as mobile phase) of 20 microliter into a HPLC fluorescence detector (Shimadzu RF 10Axl). Calibration was performed by preparation of calibration samples 2-1000 microM.

### Results

Figure 2 shows the effect of octopamine and octopamine decanoate on glycerol production in human fat suspension. Levels increased after 2 hours of incubation with octopamine (P=0.10, two tailed t-test) and reached significance for octopamine decanoate (P=0.047, two tailed t-test).

### Example 4 In vitro assay of penetration of octopamine decanoate through human skin .

4 by 4 cm square of human skin were cut and washed with PBS. Subcutaneous fat was removed and the skin was fixated over a 50 ml stirred beaker containing PBS filled so there was no air between PBS and the inside of the skin. The open surface of the skin that was exposed to the outside was 4.9 cm². To the PBS 1 ml of 0.221 mg/ml human fat that was thuraxed in PBS was added.

After stabilization of 0.5 hrs, 1 ml of hydrogel comprising 5 mg/ml octopamine decanoate was administered by gently rubbing in the skin for 1 min. The skin was covered by an inverted 20 ml beaker and left overnight for penetration and hydrolysis to occur. Samples were taken at t=0 and t=1000 min and immediately frozen at -80 degrees Celsius.

Samples were analyzed using HPLC with UV detection. A Shimadzu HPLC pump (10 ADvp) was used in combination with a valco injection valve (20 microliter loop) with a Thermo HPLC column (150 mm * 2.1 mm, BDS hypersil, C18). Octopamine was detected at 279 nm and calibration occurred by injection of standards 0-1000 microM. The mobile phase consisted of 786 mg KH₂PO₄, 500 ml ultrapure water, 15 ml MeOH, 0.5 ml acetic acid (99%) and 55.55 mg heptasulfonic acid and pumped through the system at 0.25 ml/min.

### Results

Concentrations of octopamine that were detected in the beaker 1000 min after application of the octopamine decanoate hydrogel were 0.524 microM (0.294 sem). Percentual penetration/conversion was calculated to be 0.18 +0.09 %.

From this, it follows that the octopamine decanoate prodrug penetrates the skin and is hydrolyzed after penetration by the presence of fat tissue, among which lipase.

### Example 5: In vivo effect of octopamine decanoate hydrogel treatment on waist and weight of rats.

Male wistar rats (approx. 500g) were weight daily and waistline was measured. Animals were shaven once weekly at least 12 hours before next treatment to ensure wound healing.

Animals were first treated for 1 month with carbomer hydrogel not containing prodrugs (twice daily abdominal application 3 by 3 cm), after which animals were treated with 0.5 ml carbomer hydrogel containing 5 mg/ml octopamine decanoate (application concentration 0.277 mg/cm²). Tail vena blood draws (100 microliter plasma, 5 microliter heparine 500 IE per 100microliter blood) were taken on the day -8, 21, 36 (start of compound treatment), 51 and 58. Blood was spun off (10 min 14 KRPM) and plasma was stored at -80.

After 3 weeks of treatment with octopamine decanoate (16 hrs after last application), animals were anaesthetized using isoflurane (2%, 0.8 1/min O₂) and microdialysis probes (2cm cellulose membrane, Brainlink, the Netherlands) were inserted in abdominal fat for measurement of octopamine. Probes were perfused with saline at 1.5 microliter per min and 30 min samples were collected in 300 microliter vials. Sample collection was commenced 15 min after insertion of the probe.

### Analysis

Blood and dialysate samples were analyzed for octopamine using LC-MSMS (Shimadzu 20 ADvp in conjunction with Sciex API 4000) after derivatization with SymDAQ. Briefly, 22.5 microliter samples were mixed with 0 microliter 0.5 mg/ml SymDAQ reagent and injected onto the column.Calibration was performed with samples from 0.01-8 nM.

Blood samples were analyzed for glycerol using an enzymatic kit (Sigma Aldrich) and fluorescence intensity was measured using direct flow injection (Vici valve in combination with shimadzu 10 ADvp hplc pump at 0.15 ml/min, thermo 15*2.1 C18 column prior to valve and using ultrapure water as mobile phase) of 20 microliter into a HPLC fluorescence detector (Shimadzu RF 10Axl). Calibration was performed by preparation of calibration samples 2-1000 microM.

### Results

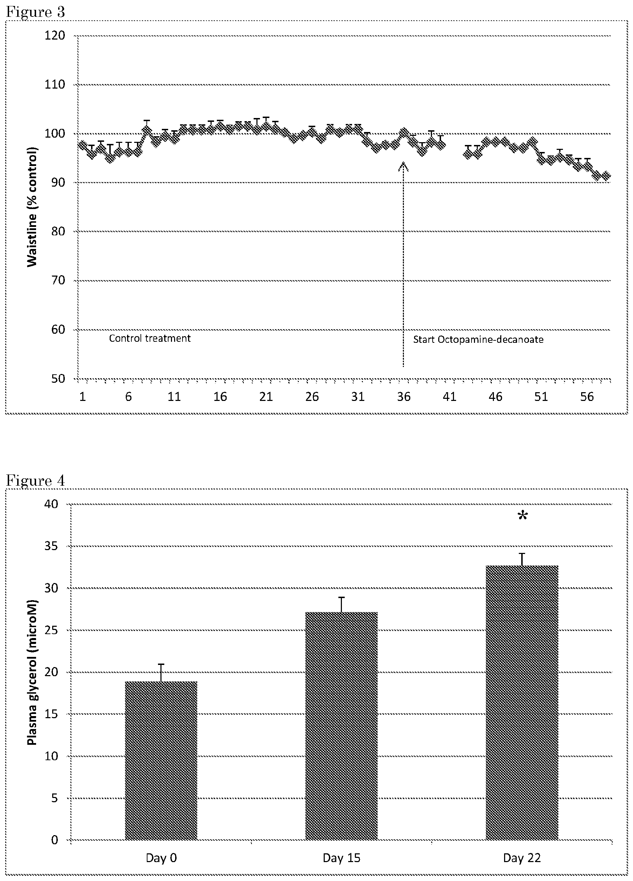

Figure 3 shows the effect of treatment of animals with control carbomer hydrogel followed by 5 mg/ml octopamine decanoate hydrogel. While animal weights remained inclining according to their growth curve, waistlines significantly reduced from initiation of application of octopamine carbomer hydrogel, reaching a reduction of about 10% after 3 weeks of treatment. Waistline was significantly reduced when compared to control treatment using a one way anova P<0.001 (1-way ANOVA RM - post hoc Bonferroni; p = 0.05).

Figure 4 shows the effect of treatment with octopamine decanoate carbomer hydrogel on plasma glycerol levels. Levels increased after 15 days (P=0.101, t-test two tailed), reaching significance after 22 days of treatment (P=0.024, t-test, two tailed).

Plasma levels of octopamine were under LLOQ (lower limit of quantification (0.1 nM) both before initiation of treatment with octopamine decanoate carbomer hydrogel, as after 15 and 22 days of treatment. This illustrates that treatment does not lead to significant systemic octopamine exposure. Subcutaneous levels of octopamine were 0.96 nM + 0.36 nM, exceeding plasma levels even 16 hours after the last application.

It follows that topical administration of an octopamine prodrug according to the invention decreases the quantity of subcutaneous fat tissue, while not leading to increased systemic concentrations of free octopamine.

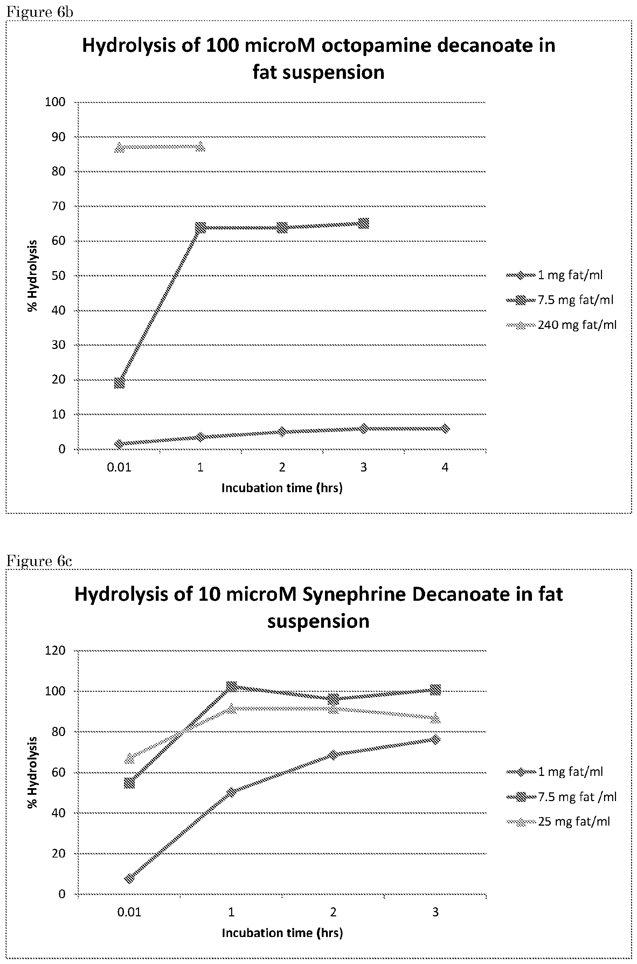

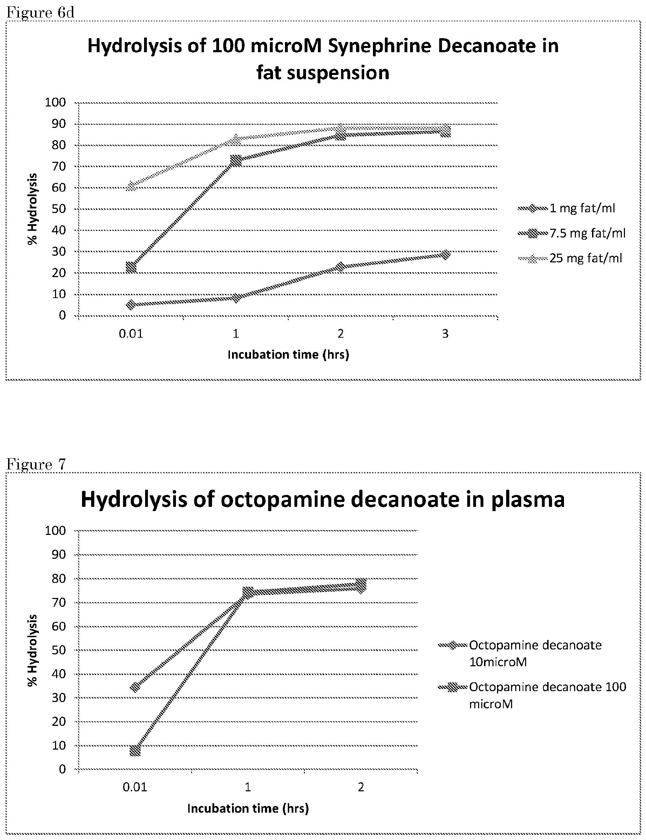

### Example 6: hydrolysis experiments (Figures 6, 7, 8, 9 and 10)

Human belly adipose tissue was obtained from obese female subjects that underwent esthetic surgery. Tissue was pottered (Potter RW 19 Nr 29795 of Janke & Kunkel KG) in PBS solution (Irvine Scientific) with 5 mM D(+)-glucose monohydrate at 530 rpm using a Teflon potter tip.

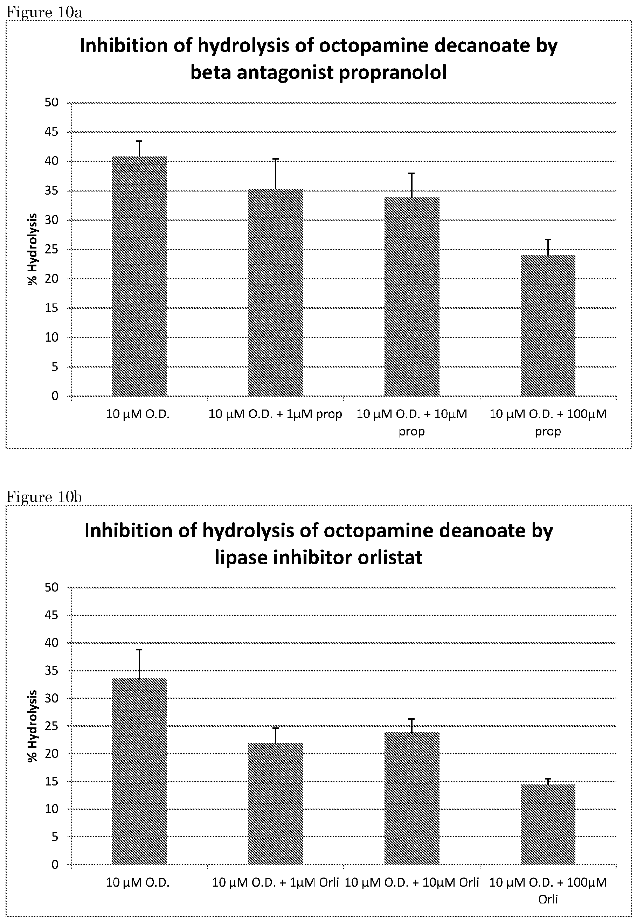

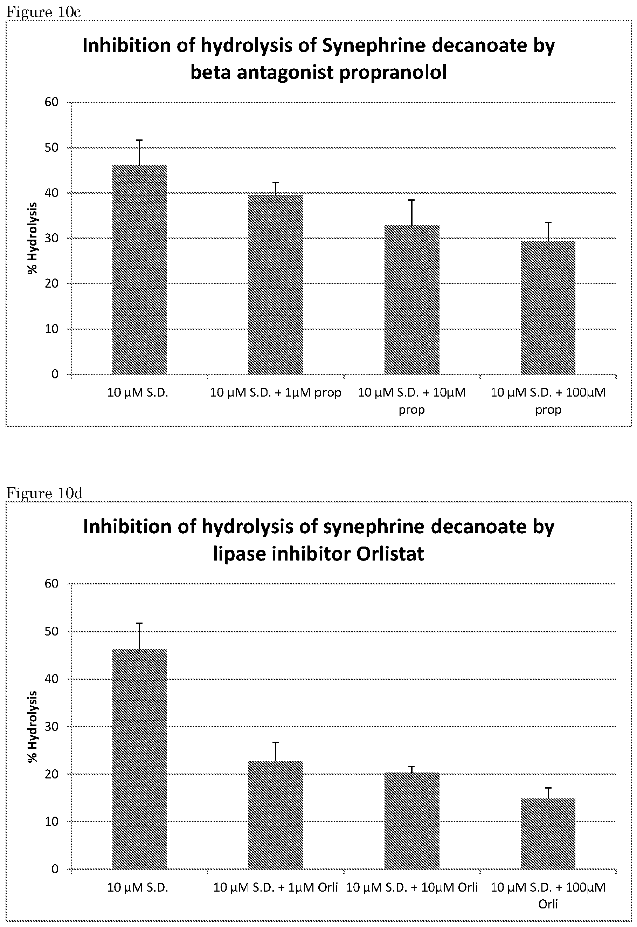

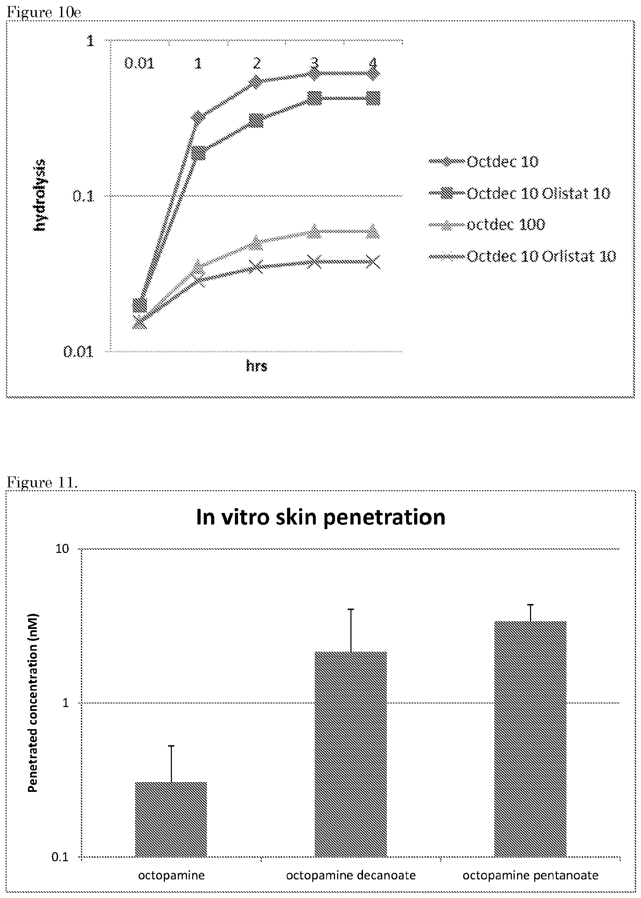

Tissue suspension (1, 7.5, 25 or 240 mg fat/ml PBS, or plasma) was stirred and 1.98 ml aliquots were transferred to polypropylene 2 ml screwcap vials (Sarstedt). Prodrug esters (octopamine decanoate and synephrine decanoate) were added at the indicated concentration, mixed and incubated at 37 °C (Figure 6). When inhibition of lipase was studied, propranolol or orlistat were added 5 minutes before addition of the prodrug esters (Figure 10).

15 minutes after addition of the prodrug, lipolysis was stopped by adding 20 microliter of 1.8% HCl. Samples were spun down (13000 rpm for 3 min at 4 °C) and supernatants were stored at -18 °C for analysis. For analysis of hydrolysis in plasma (Figure 7), 10 microliter whole blood was added to 1 ml of PBS comprising octopamine decanoate at the indicated concentration. For stability of esters, octopamine and synephrine in PBS (Figures 8 and 9), incubation was performed in PBS without fat or blood.

Samples were analyzed using HPLC UV. A Gilson 234 auto-injector and Shimadzu hplc pump (10 AdVP) was used in conjunction with a shimadzu UV (10AVp) set at 270 nm. Samples were separated using a reversed phase HPLC column (Thermo BDS Hypersil C18 150 mm X 2.1 mm, 3 micrometer). The mobile phase consisted of 1.6 g KH₂PO₄, 110 mg sodium 1-heptane sulfonate, 11 ultrapure water, 15 mL methanol and 1 ml acetic acid at a flowrate of 0.175 ml/min.

### Results

Increasing quantities of fat tissue in suspensions results in a higher hydrolysis rate of octopamine decanoate and synephrine decanoate (Figure 6 a-d). It follows that both octopamine decanoate and synephrine decanoate are hydrolyzed by the presence of fat tissue, in particular by endogenous enzymes present in fat tissue, in particular lipase.

Octopamine decanoate can also be hydrolyzed in plasma (Figure 7), which ensures that the little amount of prodrug that enters the bloodstream is rapidly converted to free octopamine, so prodrugs do not distribute throughout the body.

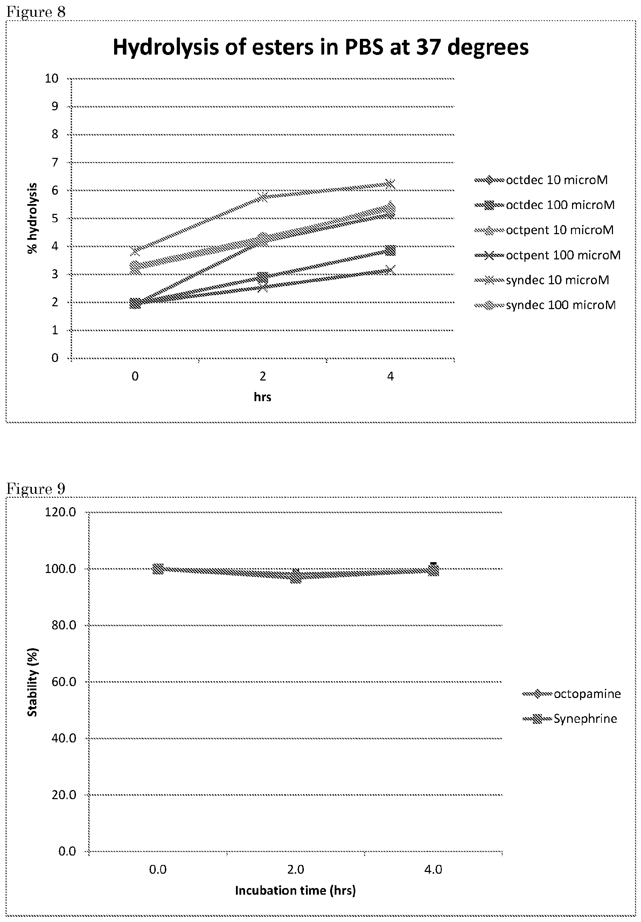

Hydrolysis of octopamine decanoate ("octdec"), octopamine pentanoate ("octpent") and synephrine decanoate ("syndec") occurs in PBS at a much lower rate than in the presence of plasma or fat tissue (Figure 8). This indicates that endogenous compounds, most likely enzymes such as lipase, are responsible for the increased hydrolysis of prodrugs of the invention into active adrenergic receptor agonists and/or antagonists.

Free octopamine or synephrine is stable in PBS (Figure 9).

The self-reinforcing, auto-catalytic effect of administration of prodrugs according to the invention can be shown as follows. If octopamine decanoate ("OD") or synephrine decanoate ("SD") is added to a fat suspension as described above, a base level of about 40 - 45 % hydrolysis is observed after 15 minutes (Figure 10).

Propranolol is a beta receptor antagonist. The beta antagonistic action of propranolol has the effect of suppressing lipase action by receptor mediation. It has been shown that addition of increasing quantities of propranolol decreases the hydrolysis of octopamine decanoate (Figure 10a) or synephrine decanoate (Figure 10c), relative to the control. Consequently, lipase from fat tissue is at least partially responsible for the hydrolysis of octopamine decanoate and synephrine decanoate in the presence of fat tissue.

This is even more apparent when adding orlistat to the suspension. Orlistat is a lipase antagonist, and consequently blocks lipase itself. It has no effect on the beta receptor-mediated stimulation or suppression of lipase. Addition of Orlistat to a suspension of octopamine decanoate (Figure 10b) or synephrine decanoate (Figure 10d) further suppresses the hydrolytic action of lipase on octopamine decanoate or synephrine decanoate. By blocking lipase itself, hydrolysis of the prodrug is suppressed to a greater extent than by blocking the beta receptor, which only has the effect of depressing lipase activation.

These results should be evaluated in context. The hydrolysis product, octopamine or synephrine, has itself the effect of stimulating the beta adrenergic receptor thereby stimulating lipase activity, as can be seen by for instance the increase in glycerol production (Figures 2 and 4). Lipase activity is also responsible for the hydrolysis of the prodrugs of the invention (Figures 6 a-d).

It follows that topical administration of prodrugs of the invention results in local hydrolysis of the prodrug to result in free octopamine or synephrine, which results in increased lipase activity. Increased lipase activity is responsible for increased hydrolysis of the prodrug, as well as increased hydrolysis of triglycerides. Thus, the prodrug of the invention is autocatalytic in driving its own hydrolysis by activation of lipase, and this activation concomitantly results in an increased hydrolysis of triglycerides (fat tissue). Thus, the action of the prodrug on lipase stimulates the lipase action on the prodrug, resulting in much increased hydrolysis of triglycerides. There is, therefore, a distinct synergy between lipase activation and hydrolysis of the prodrug.

In addition, due to the high logP of the prodrugs of the invention, the prodrugs are absorbed preferentially in fat tissue, where lipase is to be found and where triglycerides are to be hydrolyzed. This results in highly efficient hydrolysis of triglycerides, in accordance with the present claims. Thus, there is a further synergy between the autocatalytic hydrolysis mechanism described above, and the prodrug property logP, which is responsible for the partitioning of the prodrug in fat tissue.

It is postulated that it is reasonable to expect that the opposite effect, increasing the quantity of subcutaneous fat tissue by depressing lipase activity, may also occur. This is because it follows from the described experiments that suppressing lipase activity is never 100 %, so that some remant lipase activity remains. Thus, also in case of agonists or antagonists that suppress lipase activity (beta-antagonists and/or alpha agonists as described above), some lipase activity remains, allowing for hydrolysis of the prodrug and further depressing lipase activity. This would result in locally increasing the quantity of subcutaneous fat tissue, and/or reinforcing subcutaneous fat tissue.

However, as the autocatalytic effect is strongest for agonists and a antagonists that stimulate lipase activity (beta agonists and/or alpha antagonists as described above), prodrugs that stimulate lipase activity are preferred.

### Example 7: skin penetration

Human skin from obese female subjects that underwent esthetic surgery was dissected and clamped in a skin penetration chamber which allowed 6.25 cm² skin exposed over 60 ml stirred PBS (5 mM glucose) which contained 7.5 mg/ml pottered tissue for conversion of prodrugs upon penetration. 0.25 ml of a hydrogel comprising 2.75 mg/ml free octopamine, 5 mg/ml octopamine decanoate or 3.98 mg/ml octopamine pentanoate was applied to the skin once and left to penetrate for 2 hours, ensuring full hydrolysis of the prodrugs to free octopamine. The chamber was thermostated at 37 °C. Samples were drawn from the PBS pottered tissue with a 1 ml syringe and analyzed using LC-mass spectrometry. Given the full conversion, this assay evaluates the total penetration of octopamine prodrug through skin based on equal amounts of octopamine, and compares this to the penetration of an equal amount of octopamine itself.

Samples were analyzed using HPLC masspectrometry. A Shimadzu HPLC (LC20AD pump and SIL 10 ADvp injector) was used in conjunction with a sciex API 4000 Masspectrometer. The HPLC column was a Phenomenex, Synergi Max (BOL-P-RP2.5-036), 3.0 x 100mm, 2.5µm, thermostated at 35°C. The mobile phase consisted of Eluent A: 0.1% formic acid ("FA") in ultrapure water ("UP") and Eluent B: 70% acetonitrile ("ACN") + 0.1% FA at a total flow of 0.3 ml/min. The make-up flow consisted of Eluent C: 0.1% FA in ACN at 0.15 ml/min and Rinsing liquid: UP/ACN/FA = 50/50/0.1.

Separation of octopamine was accomplished by running the gradient from 0 to 40% eluent B in 4 min and than to 100% eluent B in the next 1.5 min. Gradients were maintained at 100% B for 0.5 minute.

Octopamine was determined after precipitation (25 nM octopamine-d3 in ACN/UP/FA 95%/5%/0.1%. 10 µL sample was added to 15 µL precipitation solvent and vortexed for 10 sec. Samples were centrifugated for 5 mins at 13000 rpm and 14 µL 0.1% FA in UP was added to 6 µL supernatant and vortexed for 10 sec. The autoinjector was programmed to add 20 µl 0.5 mg/ml SymDAQ reagent (online) and inject 35 µl. The SymDAQ reagent was prepared by dissolving 5 mg SymDAQ in 4.5 ml UP, 5 ml 0.25 M NaHCO₃, 0.5 ml methanol ("MeOH") and 20 microliter 2-mercapto-ethanol.

**Table 1. Settings of MSMS**

| Analyte | Q1 | Q3 | Dwell time (ms) | DP | EP | CE | CXP |
|---|---|---|---|---|---|---|---|
| Octopamine fragment 356 | 399 | 356 | 100 | 91 | 10 | 33 | 24 |
| Octopamine fragment 278 | 399 | 278 | 50 | 91 | 10 | 49 | 20 |
| Octopamine-d3 | 402 | 359 | 100 | 91 | 10 | 33 | 15 |

**Table 2. Settings of MSMS**

| Probe position | x=4 ,y=1.5 |
|---|---|
| Curtain gas (N₂) | 20 |
| CAD gas (N₂) | 8 |
| GS1 (nebulizer, zero air) | 40 |
| GS2 (zero air) | 15 |
| IS voltage | 5500 |
| ihe | On |
| Temperature | 600 |
| Resolution Q1 | Unit |
| Resolution Q3 | Unit |
| MR pause | 5 ms |
| Settling time | 2 ms |

### Results

Topical administration of a hydrogel comprising free octopamine resulted in minor penetration of octopamine through skin. Penetration of octopamine decanoate and pentanoate was about a factor 10 higher (Figure 11).

### Example 8: Skin and fat penetration assay

Cubes of human fat (5X5X5 cm) with skin attached from obese female subjects that underwent esthetic surgery was dissected at 4 °C. Cubes were transferred to containers so the skin would overlay the rim of the container. 0.25 ml of hydrogels comprising 2.75 mg/ml free octopamine, 5 mg/ml octopamine decanoate or 3.98 mg/ml octopamine pentanoate were applied once and left to penetrate for 20 hrs. Cubes were subsequently frozen at -80 °C.

Upon defrosting, skin was carefully removed taking care not to contaminate the underlying fat. The fat was dissected to yield a column of 1 X 1 cm of fat that was directly under the site of application. The column was sliced to yield 0.5 cm thick slices covering 0- 2cm fat depth under the site of application. Tissue was sonicated in 5 ml PBS (5 mM glucose), samples were spun down (13000 rpm for 30 min at 4 °C). Clear supernatant was removed with an injection needle and syringe and frozen until analysis. Analysis of octopamine was performed as described in Example 7.

### Results

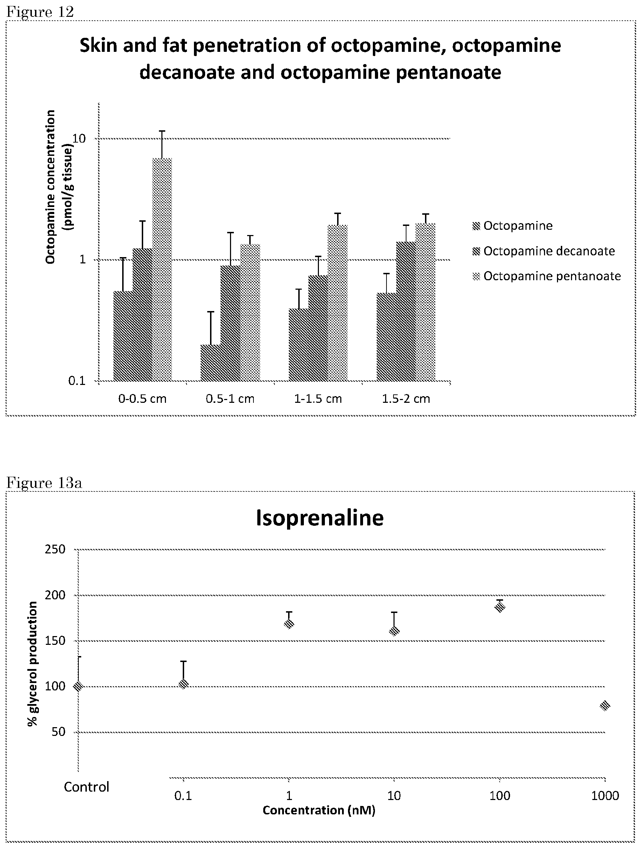

Octopamine, octopamine decanoate and octopamine pentanoate penetrate through both skin and fat tissue. Application of octopamine decanoate and pentanoate results in higher concentrations of free octopamine in all fat layers than application of a hydrogel comprising octopamine (Figure 12).

### Example 9: In vitro glycerol production assay

Human belly adipose tissue obtained from obese female subjects that underwent esthetic surgery was pottered (Potter RW 19 Nr 29795 of Janke & Kunkel KG) in PBS solution (Irvine Scientific) with 5 mM D(+)-glucose monohydrate at 530 rpm using a Teflon potter tip, to give a 250 mg/ml human fat suspension.

The fat suspension was stirred and 1.75 ml aliquots were transferred to polypropylene 2 ml screwcap vials (Sarstedt). Test compounds were added at a concentration as indicated and vials were incubated for 4 hrs at 37 °C. Vials were mixed every hour. Glycerol production in 4 hrs was calculated by analyzing glycerol content of the control (a suspension which was not incubated but instead immediately frozen) vs control samples that were incubated for 4 hours. The produced glycerol quantity was set as 100 %. The glycerol content of experimental samples was expressed as % of the control.

Experimental samples were frozen after incubation. The fat pellet was removed and upon defrosting, samples were spun down (13000 rpm for 15 min at 4 °C), and clear supernatant was pipetted off and frozen until analysis.

Glycerol was analyzed using an enzymatic kit (Glycerol Assay Kit, Sigma Aldrich). Briefly, in a 96 well plate (Corning), 100 microliter glycerol assay reaction mix was added to 10 microliter samples of supernatant and left to incubate for 20 minutes after shaking for 15 seconds. Absorbance was read by a platereader (Thermo Multiskan FC) at 570 nm. A glycerol calibration line (0.015 microM-1000 microM) was used for quantification.

### Results

Isoprenaline resulted in an increase in glycerol content at concentrations varying from 0.1 to about 800 nM (Figure 13a).

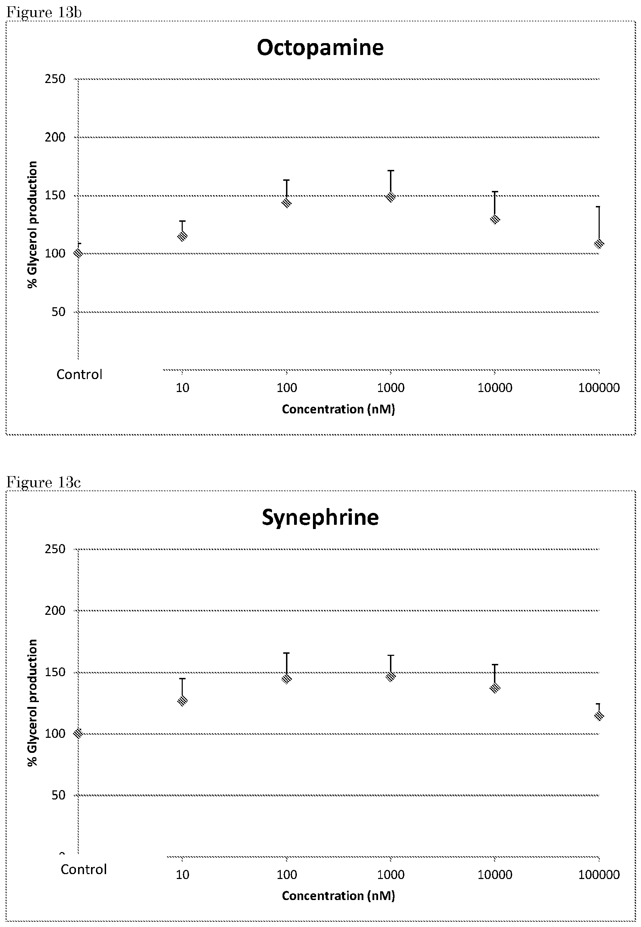

Octopamine resulted in an increase in glycerol content at concentrations varying from 0.1 to 100000 nM (Figure 13b).

Synephrine resulted in an increase in glycerol content at concentrations varying from 0.1 to at least 100000 nM (Figure 13c).

Propranolol resulted in a more or less constant glycerol content at concentrations from 0.1 to at least 1000 nM (Figure 13c). At concentrations above 1000 nM, glycerol content decreased.

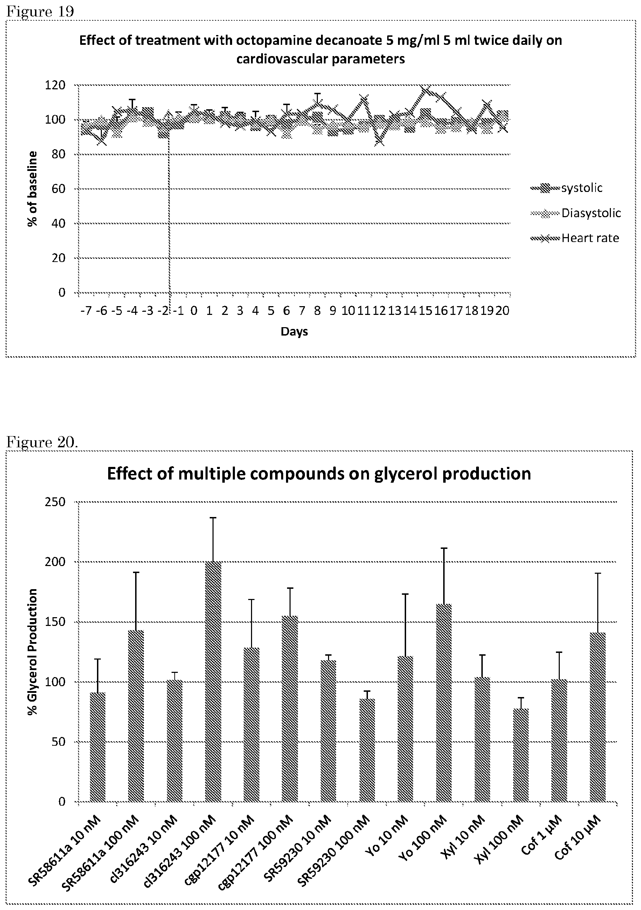

Beta 3 agonists (SR 58611A, CL 316243, CGP12177) increased glycerol production whereas beta antagonist SR 59230 reduced glycerol production (Figure 20).

Alpha 2 antagonist yohimbine increased glycerol production whereas alpha 2 agonist xylazine reduced glycerol production (Figure 20).

Phosphodiesterase inhibitor caffeine increased glycerol production (Figure 20).

From these results, it can be seen that addition of beta adrenergic receptor agonists octopamine, synephrine and isoprenaline results in hydrolysis of triglycerides to give an increased content of glycerol. This effect increases with increasing concentration of agonist, but decreases at very high concentration. It is presently assumed that desensitization of the beta receptor is responsible for the decrease in glycerol content at high concentrations of beta adrenergic receptor agonists.

Addition of the beta adrenergic receptor antagonist propranolol has the opposite effect: there is a decrease in glycerol content at concentrations above 1000 nM, and this effect increases with increasing concentration. It is assumed that antagonists with higher antagonistic activity than propranolol will display this effect at lower concentrations.

From Figure 20, it can be seen that the effects reported here also occur for other beta agonists, as well as for alpha antagonists. The opposite effect occurs for beta antagonists and alpha agonists, as has been described above.

### Example 10: Application on human volunteers

For abdominal/hip experiments 2 male volunteers 43 and 39 years old (BMI 25.5 and 28 resp.) monitored belly circumference, and skinfold at belly and hip on a daily basis (8 am mornings). Blood pressure and body weight was monitored daily. For leg experiments 1 female volunteer (42) years old (BMI 23.1) monitored leg circumference on a daily basis (8 am mornings).

### Application of hydrogel

Hydrogel was applied daily or twice daily as indicated using a 5 ml syringe to measure volume.

For abdominal experiments, the indicated volume of hydrogel was applied on the belly around the belly button in a radius of 10 cm. The same volume was applied for hip areas. For leg experiments, 2.5 ml was applied on each leg.

### Plasma sampling

After thorough washing of hands, blood was sampled using fingerprick. Blood (40 microliter) was sampled using a capillary that protruded the cap of the vial and spun down into 300 microliter vials containing 10 microliter of heparin 20 IE/ml. Plasma was pipetted off and transferred to 300 microliter vials and stored at -20 °C until analysis.

Octopamine was analyzed as described in Example 7.

### Plasma glycerol analysis

Glycerol was analyzed using a enzymatic kit (Sigma), as described above.

### Skinfold

Skinfold was assessed by measuring thickness of skin at hips and belly 4 cm from belly button using a skinfold measuring device (Vetmeter Slimguide C-120).

### Treatment regime

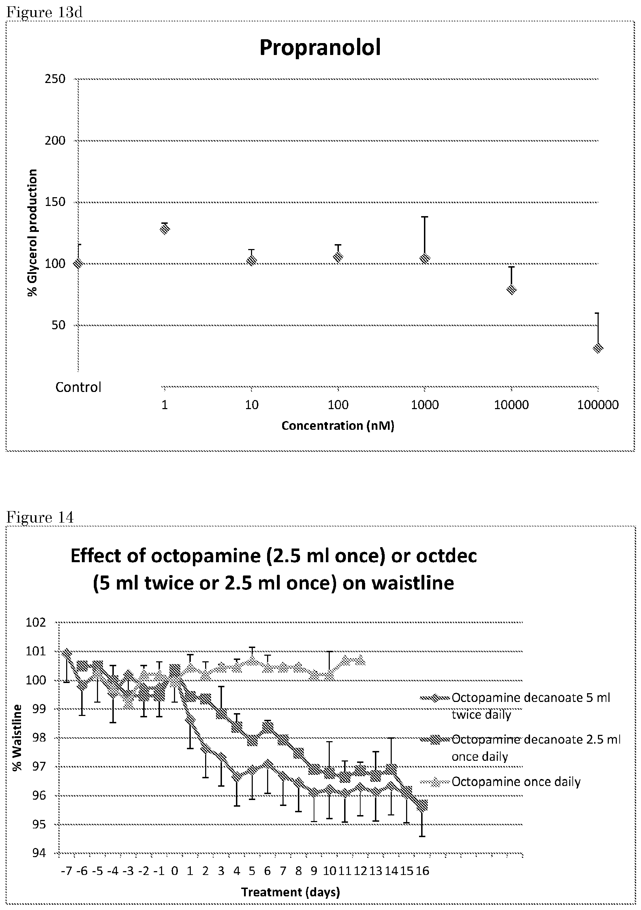

The effect of application on belly and hips of a hydrogel comprising 2.75 mg/ml free octopamine (2.5 ml, once daily), of a hydrogel comprising 5 mg/ml octopamine decanoate, (2.5 ml, once daily and 5 ml, twice daily) on waistline of humans was studied (Figure 14). Experiments are the average of two experiments (n=2).

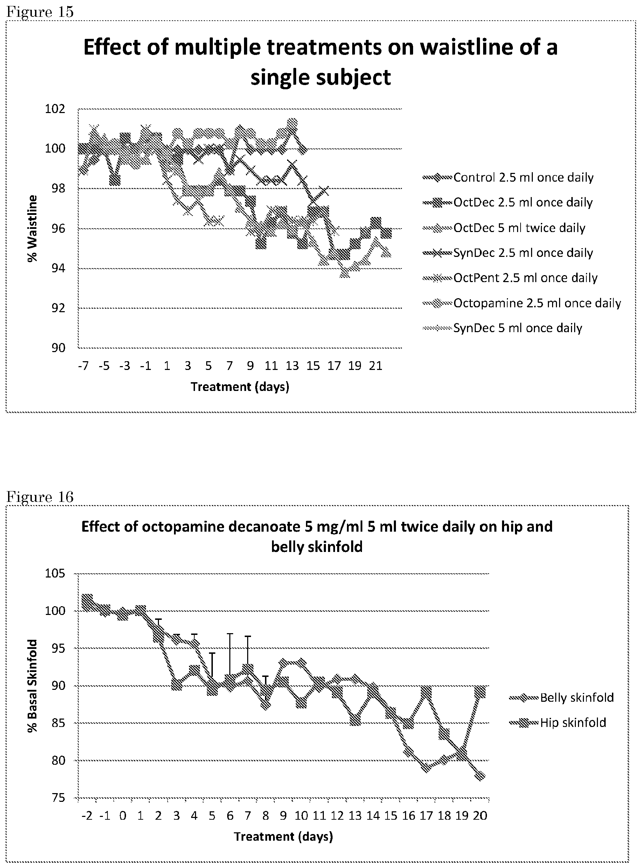

Also, the effect of application on belly and hips of control hydrogel, 2.75 mg/ml octopamine (2.5 ml, once daily), of 5 mg/ml octopamine decanoate (2.5 ml, once daily and 5 ml, twice daily) and 3.98 mg/ml octopamine pentanoate (2.5 ml, once daily) and of 5 mg/ml synephrine decanoate (2.5 ml, once daily) on waistline of a single individual was studied (Figure 15).

Also, the effect of 5 mg/ml octopamine decanoate (5 ml, twice daily) on belly and hip skinfold was studied (Figure 16). Experiments are an average of 2 or 3 runs (n=2-3).

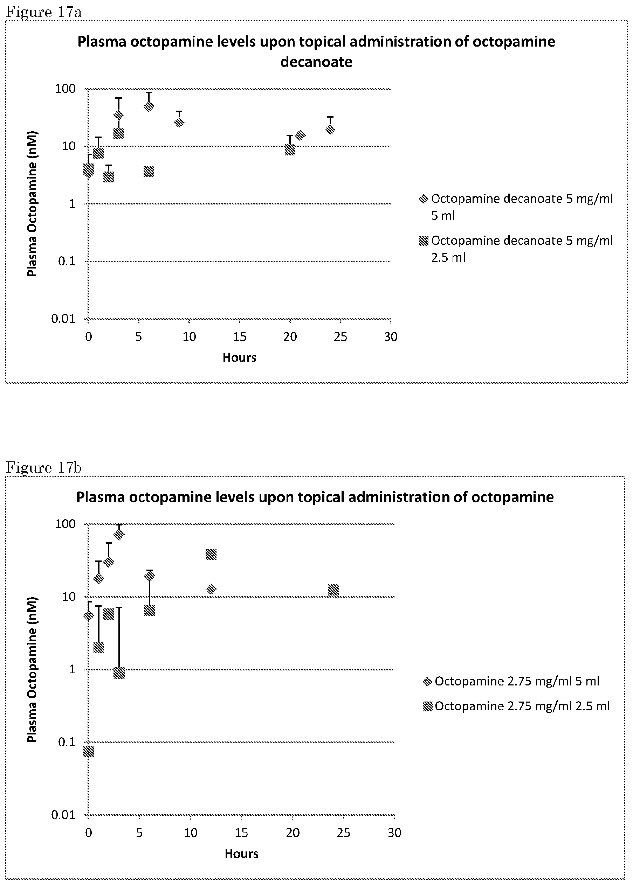

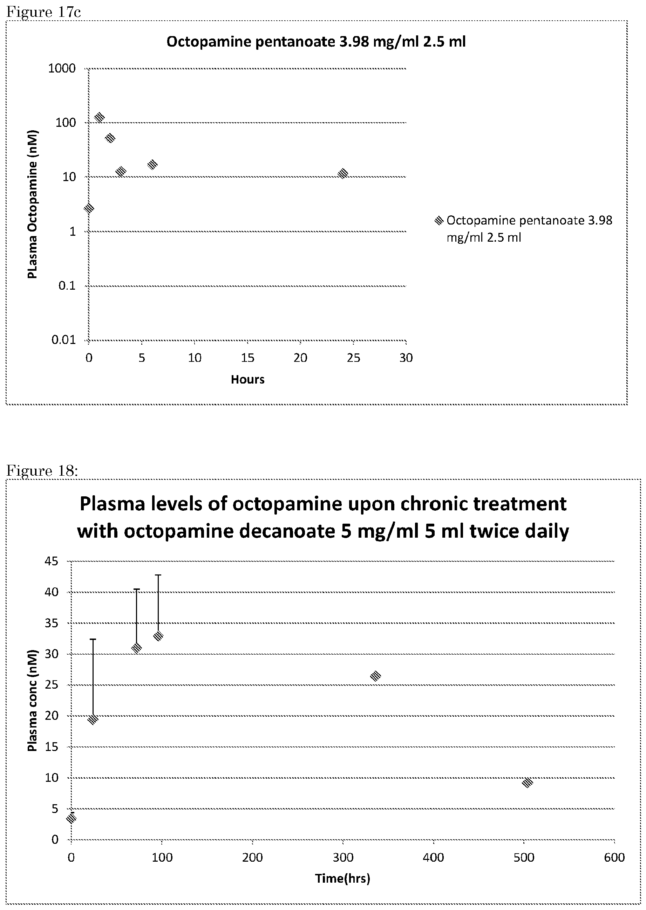

Also, plasma levels of octopamine were monitored upon administration of octopamine decanoate (5mg/ml, 2.5 ml once daily and 5 ml, twice daily; Figure 17a); of free octopamine (2.75 mg/ml, 2.5 ml, once daily and 5 ml, twice daily; Figure 17b), and of octopamine pentanoate (3.98 mg/ml, 2.5 ml, once daily); Figure 17c). Experiments are an average of one or two experiments (n=1-2).

Experiments were set up to compare equal amounts of free octopamine. Thus, the quantity of prodrug or octopamine is varied so as to provide equal amounts of free octopamine.

### Results

Topical administration of hydrogels comprising octopamine decanoate ("octdec") decreased the waistline by about 4 % after 16 days, irrespective of whether 2.5 ml once daily or 5 ml twice daily was used. Topical administration of free octopamine ("oct") in the same treatment regime and at the same molar concentration was without effect (Figure 14).

Topical administration of hydrogels comprising octopamine decanoate ("octdec"), octopamine pentanoate ("octpent" or of synephrine decanoate ("sydec") decreased the waistline by 2-5 % after 21 days. Topical administration of free octopamine ("oct") in the same treatment regime and at the same molar concentration was without effect (Figure 15).

Topical administration of hydrogels comprising octopamine decanoate reduced skinfold by 10-20 % in 20 days (Figure 16).

The treatment regimes using hydrogels comprising octopamine decanoate and pentanoate prodrugs did not result in unacceptable plasma levels of free octopamine (Figures 17a and c). Octopamine was slowly released from fat tissue to plasma, and no side effects were reported. The administration of prodrugs resulted in acceptable systemic concentrations of free octopamine, which was also the case for adinistration of a hydrogel comprising free octopamine (Figure 17b). However, the hydrogel comprising free octopamine dis not result in a decrease of subcutaneous fat tissue (Figure 15).

During chronic treatment with a hydrogel comprising octopamine decanoate (5 mg/ml, 5 ml, twice daily), plasma levels of octopamine remained at acceptable values. No side effects were reported (Figure 18).

Blood pressure (systole ("syst") and diastole ("dia")) and heart rate ("HR") remained normal during these experiments (Figure 19), and no side effects were reported.

### Example 11: removal of cellulite

A hydrogel comprising 3.98 mg/ml octopamine pentanoate (5 ml) was applied once daily on a human subject in an area with moderate cellulite. After three days the cellulite was noticeably decreased.

### Figures

Figure 1: hydrolysis of octopamine decanoate ("octdec") in phosphate buffered saline ("PBS"), PBS/rat fat suspension or PBS/human fat suspensions (diamond n=3, square n=2, triangles n=2).
Figure 2:effect of incubation of PBS/human fat suspension with octopamine or octopamine decanoate on glycerol production
Figure 3: effect of treatment of rats with carbomer hydrogel (0.5 ml on 3X3 cm) followed by 5 mg/ml octopamine decanoate in carbomer hydrogel (0.5 ml on 3X3 cm) on waistline. Treatment was twice daily at 9 am and 4 pm (n=4 each).
Figure 4: effect of treatment of rats with carbomer hydrogel (0.5 ml on 3X3 cm) followed by 5 mg/ml octopamine decanoate in carbomer hydrogel (0.5 ml on 3X3 cm) on plasma glycerol levels (n=4 each).
Figure 5: potential synthesis route toward octopamine decanoate.
Figure 6: Hydrolysis of 10 and 100 microM octopamine decanoate (Figure 6 a & b) and 10 and 100 microM synephrine decanoate (Figure 6 c & d) in vitro at different concentrations of human fat.
Figure 7: Hydrolysis of octopamine decanoate in plasma.
Figure 8: Hydrolysis of octopamine decanoate, octopamine pentanoate and synephrine decanoate in PBS at 37 degrees.
Figure 9: Stability of octopamine and synephrine in PBS at 37 degrees. Experiments are n=2.
Figure 10: Inhibition of hydrolysis in fat suspension of octopamine decanoate 10 microM and synephrine decanoate 10 microM by beta antagonist propranolol (fig 10a and c) and lipase inhibitor orlistat (Figure 10 b and d). Experiments are n=4-8. The same data can be represented in time (Figure 10e)
Figure 11: In vitro skin penetration through human skin after application of 0.25 ml of octopamine hydrogel 2.75 mg/ml, octopamine decanoate 5 mg/ml and octopamine pentanoate 3.98 mg/ml. Penetration is concentration of 60 ml perfusion bath below the skin, 2 hrs after application. Experiments are n=2-3.
Figure 12: Tissue concentration of octopamine in layers at increasing depth of human subcutaneous fat after application of 0.25 ml of octopamine (2.75 mg/ml), octopamine decanoate (5 mg/ml) and octopamine pentanoate (3.98 mg/ml), 20 hrs after application.
Figure 13: Effect of Isoprenaline (Figure 13a), octopamine (Figure 13b), synephrine (Figure 13c) and propranolol (Figure 13d) on glycerol formation in a 250 mg/ml human fat suspension during 4 hrs at 37 degrees Celsius. Experiments are n=4-20.
Figure 14: Effect of application on belly and hips of a hydrogel comprising 2.75 mg/ml free octopamine (2.5 ml, once daily), of a hydrogel comprising 5 mg/ml octopamine decanoate, (2.5 ml, once daily) and a hydrogel comprising 5 mg/ml octopamine decanoate (5 ml, twice daily) on waistline of humans. Experiments are the average of two experiments (n=2).
Figure 15: Effect of application on belly and hips of control hydrogel without agonist or antagonist (2.5 ml, once daily), 2.75 mg/ml octopamine (2.5 ml, once daily), of 5 mg/ml octopamine decanoate ("OctDec", 2.5 ml, once daily and 5 ml, twice daily) and 3.98 mg/ml octopamine pentanoate ("OctPent", 2.5 ml, once daily) and of 5 mg/ml synephrine decanoate ("SynDec", 2.5 ml, once daily and 5 ml, once daily) on waistline of a single individual.
Figure 16: Effect of a hydrogel comprising 5 mg/ml octopamine decanoate (5 ml, twice daily) on belly and hip skinfold. Experiments are an average of 2 or 3 runs (n=2-3).
Figure 17: Plasma levels of octopamine upon single administration of hydrogels comprising octopamine decanoate (5mg/ml, 2.5 ml and 5 ml; Figure 17a); free octopamine (2.75 mg/ml, 2.5 ml and 5 ml; Figure 17b), and octopamine pentanoate (3.98 mg/ml 2.5 ml; Figure 17c). Experiments are an average of one or two experiments (n=1-2).
Figure 18: Plasma levels of free octopamine 12 hours after application, during chronic treatment with a hydrogel comprising 5 mg/ml octopamine decanoate (5 ml, twice daily). Experiments are an average of 1 or two runs (n=1-2).
Figure 19: Systole, diastole and heart rate upon application of a hydrogel comprising 5 mg/ml octopamine decanoate (5 ml, twice daily) on belly and hips. Data represent an average of two persons.
Figure 20: Effect of beta-3 agonists (SR 58611A, CL 316243, CGP12177), beta antagonist (SR 59230A), the alpha-2 antagonist Yohimbine ("Yo"), alpha 2 agonist Xylazine ("Xyl") and phosphodiesterase inhibitor caffeine ("Cof") on glycerol formation in a 250 mg/ml human fat suspension during 4 hrs at 37 degrees Celsius. Experiments are n=3-4.
Figure 21: potential synthesis route toward synephrine decanoate.
Figure 22: general synthetic approach toward prodrug esters of the invention, wherein R₁ₐ and R_{1b} is H or OH, and at least one of R₁ₐ and R_{1b} is OH, R₂ is H or methyl, X is a leaving group, preferably chloride, bromide or iodide, R₃ is benzyl or alkyl (preferably methyl or isopropyl), R₄ is a C1-C31 alkyl group to provide the C2-C32 alkyl ester as defined above, and wherein at least of R₅ₐ and R_{5b} is R₄CO.
Figure 23: General route for the synthesis of ester prodrugs, wherein 10 is an agonist or antagonist for an adrenergic receptor as defined elsewhere, which has a free OH-group, which free OH-group is preferably a benzylic or phenolic OH- group; and wherein 11 is an acylating agent, preferably an acid halide or an anhydride, wherein LG is a leaving group, preferably selected from a halide (preferably chloride), or a carboxylate, and wherein HM is a hydrolyzable moiety as defined elsewhere.
Figure 24: A general route for formation of an amide prodrug **15,** wherein **13** is an agonist or antagonist for an adrenergic receptor as described elsewhere, which has a free amine group comprising at least one amine hydrogen, which free amine group is preferably a primary alkyl amine, wherein R" is selected from H or a C1-C8 linear, branched or cyclic alkyl group such as methyl, ethyl or isopropyl, preferably H, and wherein preferably, free OH-groups, more preferably phenolic or benzylic free OH-groups, are protected by a suitable protecting group, and wherein **14** is a hydrolyzable moiety as defined elsewhere functionalized with a carboxylic acid group, and wherein the coupling agent can be any known coupling agent, such as for instance DCC, EDCI, HATU or HBTU.
Figure 25: A general route for formation of a carbamate prodrug **18,** wherein **16** is an agonist or antagonist for an adrenergic receptor as described elsewhere, which has a free OH-group, which free OH-group is preferably a benzylic or phenolic OH- group; and wherein **17** is a hydrolyzable moiety as defined elsewhere, functionalized with an isocyanate

## Claims

1. A pharmaceutical composition for topical administration, comprising a prodrug for an agonist and/or an antagonist for an adrenergic receptor, wherein the prodrug is an ester, which prodrug comprises said agonist or antagonist and a hydrolyzable moiety, wherein the prodrug has an octanol/water partition coefficient of at least 0, for use in a therapeutic method of shaping a mammalian body by modulation of subcutaneous fat tissue.

2. A pharmaceutical composition for use according to claim 1, wherein the modulation occurs at the site of topical administration.

3. A pharmaceutical composition for use according to claim 1 or 2, wherein modulation comprises decreasing the quantity of subcutaneous fat tissue, increasing the quantity of subcutaneous fat tissue, or reinforcing subcutaneous fat tissue, and wherein preferably, modulation comprises decreasing the quantity of subcutaneous fat tissue.

4. A pharmaceutical composition for use according to any of claims 1-3, wherein the prodrug has an octanol/water partition coefficient of at least 2.3.

5. A pharmaceutical composition for use according to any of claims 1-4, wherein the agonist is an agonist for a beta adrenergic receptor ("beta-agonist") or an agonist for an alpha-adrenergic receptor ("alpha-agonist"), and/or wherein the antagonist is an antagonist for the beta-adrenergic receptor ("beta-antagonist") or an antagonist for the alpha-adrenergic receptor ("alpha-antagonist").

6. A pharmaceutical composition for use according to claim 5, wherein
• the beta-agonist is octopamine (ortho-, meta- or para-octopamine, preferably para-octopamine), synephrine (ortho-, meta- or para-synefrine, preferably para-synephrine), norepinephrine, epinephrine, ephedrine, phenylpropanolamine, tyramine, epinine, phenylethanolamine, beta-phenylethylamine, hordenine, isopropylnorsynephrine, N-methyltyramine, salbutamol, levosalbutamol, terbutaline, pirbuterol, procaterol, clenbuterol, metaproterenol, fenoterol, bitolterol, ritodrine, isoprenaline, salmeterol, formoterol, bambuterol, clenbuterol, olodaterol, indacaterol, Amibegron (SR-58611A), CL 316,243, L-742,791, L-796,568, LY-368,842, Mirabegron (YM-178), Ro40-2148, CGP12177, Solabegron (GW-427,353), BRL 37,344;
• the alpha antagonist is Aripiprazole, Asenapine, Atipamezole, Cirazoline, Clozapine, Efaroxan, Idazoxan, Lurasidone, Melperone, Mianserin, Mirtazapine, Napitane, Olanzapine, Paliperidone, Risperidone, Phenoxybenzamine, Phentolamine, Piribedil, Rauwolscine, Risperidone, Rotigotine, Quetiapine, Norquetiapine, Setiptiline, Tolazoline, Yohimbine, Ziprasidone or Zotepine;
• the beta-antagonist is Carteolol, Nadolol, Penbutolol, Pindolol, Propranolol, Sotalol, Timolol, Acebutolol, Atenolol, Betaxolol, Bisoprolol, Celiprolol, Esmolol, Metoprolol, Nebivolol, Bucindolol, Carvedilol, Labetolol, preferably Penbutolol, Pindolol, Propranolol, Atenolol, Metoprolol L-748,328, L-748,337, SR 59230A;
• the alpha-agonist is 4-NEMD, 7-Me-marsanidine, Agmatine, Apraclonidine, Brimonidine, Clonidine, Detomidine, Dexmedetomidine, Fadolmidine, Guanabenz, Guanfacine, Lofexidine, Marsanidine,Medetomidine, Methamphetamine, Mivazerol, Rilmenidine, Romifidine, Talipexole, Tizanidine, Tolonidine, Xylazine, Xylometazoline, TDIQ.

7. A pharmaceutical composition for use according to any of claims 1 - 6, wherein the ester is a C2-C32 alkyl ester, preferably a butanoate, pentanoate, heptanoate, octanoate or decanoate ester.

8. A pharmaceutical composition for use according to any of claims 1 - 7, wherein the prodrug is present in the composition at a concentration of 0.001-1000 mg/ml.

9. A pharmaceutical composition for use according to any of claims 1 - 8, wherein the pharmaceutical composition is a cream, foam, gel, lotion, ointment, patch, paste, solution or spray.

10. A pharmaceutical composition for use according to any of claims 1-9, wherein the prodrug is a beta-agonist, preferably octopamine or synefrine, preferably p-octopamine or p-synefrine.

11. A pharmaceutical composition for use according to any of the previous claims, further comprising a phosphodiesterase inhibitor and/or an adenyl cyclase stimulator.

12. A cosmetic method of shaping a mammalian body by locally modulating subcutaneous fat tissue, comprising topically administering a pharmaceutical composition as defined in any of claims 1 - 11.

13. A method according to claim 12, wherein the prodrug is administered at a dosage of 0.001-1000 mg/cm².

14. A method according to claim 12 or 13, wherein the local modulation comprises decreasing the quantity of subcutaneous fat tissue, increasing the quantity of subcutaneous fat tissue, or reinforcing subcutaneous fat tissue.

15. A method according to claim 14, wherein the method locally removes wrinkles or cellulite.

16. A prodrug for octopamine, wherein the prodrug is an ester comprising octopamine and a hydrolyzable moiety, wherein the prodrug has an octanol/water partition coefficient of at least 0, preferably at least 2.3.

17. A prodrug according to claim 16 for medical use, preferably for use in a method of shaping a mammalian body by modulation of subcutaneous fat tissue.

18. A method of making a prodrug for octopamine, comprising esterifying octopamine with an acylating agent.

## Patentansprüche

1. Pharmazeutische Zusammensetzung zur topischen Verabreichung, umfassend ein Pro-Pharmakon für einen Agonisten und/oder einen Antagonisten für einen adrenergen Rezeptor, wobei das Pro-Pharmakon ein Ester ist, das Pro-Pharmakon den Agonisten oder Antagonisten und eine hydrolysierbare Einheit umfasst, wobei das Pro-Pharmakon einen Octanol/Wasser-Verteilungskoeffizienten von wenigstens 0, zur Verwendung in einem therapeutischen Verfahren zum Formen eines Säugetierkörpers durch Modulation von subkutanem Fettgewebe hat.

2. Pharmazeutische Zusammensetzung zur Verwendung nach Anspruch 1, wobei die Modulation an der Stelle der topischen Verabreichung erfolgt.

3. Pharmazeutische Zusammensetzung zur Verwendung nach Anspruch 1 oder 2, wobei die Modulation Verringern der Menge von subkutanem Fettgewebe, Erhöhen der Menge von subkutanem Fettgewebe, oder Verstärken von subkutanem Fettgewebe umfasst, und wobei vorzugsweise Modulation Verringern der Menge von subkutanem Fettgewebe umfasst.

4. Pharmazeutische Zusammensetzung zur Verwendung nach einem der Ansprüche 1 - 3, wobei das Pro-Pharmakon einen Octanol/Wasser-Verteilungskoeffizienten von wenigstens 2,3 hat.

5. Pharmazeutische Zusammensetzung zur Verwendung nach einem der Ansprüche 1 - 4, wobei der Agonist ein Agonist für einen beta-adrenergen Rezeptor ("Beta-Agonist") oder ein Agonist für einen alpha-adrenergen Rezeptor ("alpha-Agonist") ist, und/oder wobei der Antagonist ein Antagonist für den beta-adrenergen Rezeptor ("beta-Antagonist") oder ein Antagonist für den alpha-adrenergen Rezeptor ("alpha-Antagonist") ist.

6. Pharmazeutische Zusammensetzung zur Verwendung nach Anspruch 5, wobei
• der Beta-Agonist Octopamin (ortho-, meta- oder para-Octopamin, vorzugsweise para-Octopamin), Synephrin (ortho-, meta- oder para-Synefrin, vorzugsweise para-Synephrin), Noradrenalin, Adrenalin, Ephedrin, Phenylpropanolamin, Tyramin, Epinin, Phenylethanolamin, Beta-Phenylethylamin, Hordenin, Isopropylnorsynephrin, N-Methyltyramin, Salbutamol, Levosalbutamol, Terbutalin, Pirbuterol, Procaterol, Clenbuterol, Metaproterenol, Fenoterol, Bitolterol, Ritodrin, Isoprenalin, Salmeterol, Formoterol, Bambuterol, Clenbuterol, Olodaterol, Indacaterol, Amibegron (SR-58611A), CL 316,243, L-742,791, L- 796,568, LY-368,842, Mirabegron (YM-178), Ro40-2148, CGP12177, Solabegron (GW-427,353), BRL 37,344 ist;
• der Alpha-Antagonist Aripiprazol, Asenapin, Atipamezol, Cirazolin, Clozapin, Efaroxan, Idazoxan, Lurasidon, Melperon, Mianserin, Mirtazapin, Napitan, Olanzapin, Paliperidon, Risperidon, Phenoxybenzamin, Phentolamin, Piribedil, Rauwolscin, Risperidon, Rotigotin, Quetiapin, Norquetiapin, Setiptilin, Tolazolin, Yohimbin, Ziprasidon oder Zotepin ist;
• der Beta-Antagonist Carteolol, Nadolol, Penbutolol, Pindolol, Propranolol, Sotalol, Timolol, Acebutolol, Atenolol, Betaxolol, Bisoprolol, Celiprolol, Esmolol, Metoprolol, Nebivolol, Bucindolol, Carvedilol, Labetolol, vorzugsweise Penbutolol, Pindolol, Propranolol, Atenolol, Metoprolol L-748,328, L-748,337, SR 59230A ist;
• der alpha-Agonist 4-NEMD, 7-Me-Marsanidin, Agmatin, Apraclonidin, Brimonidin, Clonidin, Detomidin, Dexmedetomidin, Fadolmidin, Guanabenz, Guanfacin, Lofexidin, Marsanidin, Medetomidin, Methamphetamin, Mivazerol, Rilmenidin, Romifidin, Talipexol, Tizanidin, Tolonidin, Xylazin, Xylometazolin, TDIQ ist.

7. Pharmazeutische Zusammensetzung zur Verwendung nach einem der Ansprüche 1 - 6, wobei der Ester ein C2-C32 Alkylester, vorzugsweise ein Butanoat-, Pentanoat-, Heptanoat-, Octanoat- oder Decanoatester ist.

8. Pharmazeutische Zusammensetzung zur Verwendung nach einem der Ansprüche 1 - 7, wobei das Pro-Pharmakon in der Zusammensetzung in einer Konzentration von 0,001 bis 1000 mg/ml vorliegt.

9. Pharmazeutische Zusammensetzung zur Verwendung nach einem der Ansprüche 1 - 8, wobei die pharmazeutische Zusammensetzung eine Creme, Schaum, Gel, Lotion, Salbe, Pflaster, Paste, Lösung oder Spray ist.

10. Pharmazeutische Zusammensetzung zur Verwendung nach einem der Ansprüche 1 - 9, wobei das Pro-Pharmakon ein Beta-Agonist, vorzugsweise Octopamin oder Synefrin, vorzugsweise p-Octopamin oder p-Synefrin, ist.

11. Pharmazeutische Zusammensetzung zur Verwendung nach einem der vorhergehenden Ansprüche, ferner umfassend einen Phosphodiesterase-Inhibitor und/oder einen Adenylcyclase-Stimulator.

12. Kosmetisches Verfahren zum Formen eines Säugetierkörpers durch lokales Modulieren von subkutanem Fettgewebe, umfassend topisches Verabreichen einer pharmazeutischen Zusammensetzung wie definiert in einem der Ansprüche 1 - 11.

13. Verfahren nach Anspruch 12, wobei das Pro-Pharmakon in einer Dosierung von 0,001-1000 mg/cm² verabreicht wird.

14. Verfahren nach Anspruch 12 oder 13, wobei die lokale Modulation Verringern der Menge von subkutanem Fettgewebe, Erhöhen der Menge von subkutanem Fettgewebe, oder Verstärken von subkutanem Fettgewebe umfasst.

15. Verfahren nach Anspruch 14, wobei das Verfahren lokal Falten oder Cellulite entfernt.

16. Pro-Pharmakon für Octopamin, wobei das Pro-Pharmakon ein Ester ist, umfassend Octopamin und eine hydrolysierbare Einheit, wobei das Pro-Pharmakon einen Octanol/Wasser-Verteilungskoeffizienten von wenigstens 0, vorzugsweise wenigstens 2,3 hat.

17. Pro-Pharmakon nach Anspruch 16 zur medizinischen Verwendung, vorzugsweise zur Verwendung in einem Verfahren zum Formen eines Säugetierkörpers durch Modulation von subkutanem Fettgewebe.

18. Verfahren zur Herstellung eines Pro-Pharmakon für Octopamin, umfassend Verestern von Octopamin mit einem Acylierungsmittel.

## Revendications

1. Composition pharmaceutique pour administration topique, comprenant un promédicament pour un agoniste et/ou un antagoniste d'un récepteur adrénergique, dans laquelle le promédicament est un ester, lequel promédicament comprenant ledit agoniste ou antagoniste et une fraction hydrolysable, dans laquelle le promédicament contient un coefficient de partition octanol/eau d'au moins 0, destiné à être utilisé dans un procédé thérapeutique de mise en forme d'un corps de mammifère par modulation de tissu adipeux sous-cutané.

2. Composition pharmaceutique à utiliser selon la revendication 1, dans laquelle la modulation a lieu au niveau du site d'administration topique.

3. Composition pharmaceutique à utiliser selon la revendication 1 ou 2, dans laquelle la modulation comprend une diminution de la quantité de tissu adipeux sous-cutané, une augmentation de la quantité de tissu adipeux sous-cutané ou un renforcement de tissu adipeux sous-cutané, et dans lequel, de préférence, la modulation comprend une diminution de la quantité de tissu adipeux sous-cutané.

4. Composition pharmaceutique à utiliser selon l'une quelconque des revendications 1 à 3, dans laquelle le promédicament a un coefficient de partition octanol/eau d'au moins 2,3.

5. Composition pharmaceutique à utiliser selon l'une quelconque des revendications 1 à 4,
dans laquelle l'agoniste est un agoniste pour un récepteur bêta-adrénergique (« bêta-agoniste ») ou un agoniste pour un récepteur alpha-adrénergique (« alpha-agoniste »), et/ou
dans lequel l'antagoniste est un antagoniste du récepteur bêta-adrénergique (« bêta-antagoniste ») ou un antagoniste du récepteur alpha-adrénergique (« alpha-antagoniste »).

6. Composition pharmaceutique à utiliser selon la revendication 5,
dans laquelle le bêta-agoniste est l'octopamine (ortho, méta ou para-octopamine, de préférence para-octopamine), la synéphrine (ortho, méta ou para-synéphrine, de préférence para-synéphrine), la norépinéphrine, l'épinéphrine, l'éphédrine, la phénylpropanolamine, la tyramine, la phényléthanolamine, la bêta-phényléthylamine, l'hordénine, l'isopropylnorsynéphrine, la N-méthyltyramine, le salbutamol, le lévosalbutamol, la terbutaline, le pirbutérol, le procatérol, le clenbutérol, le métaproténérol, le fénoténérol, le bitoltérol, la ritodrine, l'isoprénaline, le salmétérol, le formotérol, le bambutérol, le clenbutérol, l'olodatérol, l'indacatérol, l'Amibegron (SR-58611A), CL 316,243, L-742,791, L796,568, LY-368,842, le Mirabégron (YM-178), Ro40-2148, CGP12177, Solabégron (GW-427.353), BRL 37,344 ;
l'alpha antagoniste est l'Aripripazole, l'Asénapine, l'Atipamézole, la Cirazoline, la Clozapine, l'Efaxoran, l'Idazoxan, la Lurasidone, la Melpérone, la Miansérine, la Mirtazapine, la Napitane, l'Olazanpine, la Palipéridone, la Rispéridone, la Phénoxybenzamine, la Phentolamine, le Piribédil, la Rauwolscine, la Rispéridone, la Rotigotine, la Quétiapine, la Norquétiapine, la Setiptiline, la Tolazoline, la Yohimbine, la Ziprasidone ou la Zotépine ;
le bêta-antagoniste est le Cartéolol, le Nadolol, le Penbutolol, le Pindolol, le Propranolol, le Sotalol, le Timolol, l'Acébutolol, l'Aténolol, le Bétaxolol, le Bisoprolol, le Céliprolol, l'Esmolol, le Métoprolol, le Nébivolol, le Bucindolol, le Carvédilol, le Labétolol, de préférence le Penbutolol, le Pindolol, le Propranolol, l'Aténolol, le Métoprolol L-748,328, L-748,337, SR 59230A ;
l'alpha-agoniste est 4-NEMD, la 7-Me-marsanidine, l'Agmatine, l'Apraclonidine, la Brimonidine, la Clonidine, la Détomidine, la Dexmédétomidine, la Fadolmidine, le Guanabenz, la Guanfacine, la Loféxidine, la Marsanidine, la Médétomidine, la Mététamine, la Méthamphétamine, le Mivazérol, la Rilménidine, la Romifidine, le Talipexole, la Tizanidine, la Tolonidine, la Xylazine, la Xylométazoline, TDIQ.

7. Composition pharmaceutique à utiliser selon l'une quelconque des revendications 1 à 6,
dans lequel l'ester est un ester alkylique en C₂-C₃₂, de préférence un ester de butanoate, pentanoate, heptanoate, octanoate ou décanoate.

8. Composition pharmaceutique à utiliser selon l'une quelconque des revendications 1 à 7, dans laquelle le promédicament est présent dans la composition à une concentration de 0,001 à 1000 mg/ml.

9. Composition pharmaceutique à utiliser selon l'une quelconque des revendications 1 à 8, dans laquelle la composition pharmaceutique est une crème, une mousse, un gel, une lotion, une pommade, un timbre, une pâte, une solution ou un spray.

10. Composition pharmaceutique à utiliser selon l'une quelconque des revendications 1 à 9, dans laquelle le promédicament est un bêta-agoniste, de préférence de l'octopamine ou de la synéfrine, de préférence de la p-octopamine ou de la p-synéfrine.

11. Composition pharmaceutique à utiliser selon l'une quelconque des revendications précédentes, comprenant en outre un inhibiteur de phosphodiestérase et/ou un stimulateur d'adénylcyclase.

12. Procédé cosmétique de mise en forme d'un corps de mammifère en modulant localement le tissu adipeux sous-cutané, comprenant l'administration topique d'une composition pharmaceutique telle que définie dans l'une quelconque des revendications 1 à 11.

13. Procédé selon la revendication 12, dans lequel le précurseur de médicament est administré à une dose de 0,001 à 1000 mg/cm².

14. Procédé selon la revendication 12 ou 13, dans lequel la modulation locale comprend une diminution de la quantité de tissu adipeux sous-cutané, une augmentation de la quantité de tissu adipeux sous-cutané ou un renforcement de tissu adipeux sous-cutané.

15. Procédé selon la revendication 14, dans lequel le procédé élimine localement les rides ou la cellulite.

16. Promédicament pour l'octopamine, dans lequel le promédicament est un ester comprenant de l'octopamine et un fragment hydrolysable, dans lequel le promédicament a un coefficient de partition octanol/eau d'au moins 0, de préférence d'au moins 2,3.

17. Promédicament selon la revendication 16 à usage médical, de préférence à utiliser dans un procédé de mise en forme d'un corps de mammifère par modulation de tissu adipeux sous-cutané.

18. Procédé de fabrication d'un promédicament pour l'octopamine, comprenant l'estérification de l'octopamine avec un agent acylant.
